(19) Europäisches Patentamt
European Patent Office
Office européen des brevets

(11) Veröffentlichungsnummer: **0 167 028**
**B1**

(12) # EUROPÄISCHE PATENTSCHRIFT

(45) Veröffentlichungstag der Patentschrift: **09.03.88**

(21) Anmeldenummer: **85107170.4**

(22) Anmeldetag: **11.06.85**

(51) Int. Cl.⁴: **C 07 D 403/04**, C 07 D 413/04, C 07 D 417/04, C 07 D 401/04, C 07 D 405/04, A 01 N 43/56, C 07 D 231/38, C 07 D 231/40

(54) **5-Amino-4-heterocyclyl-1-phenylpyrazole.**

(30) Priorität: **22.06.84 DE 3423101**

(43) Veröffentlichungstag der Anmeldung: **08.01.86 Patentblatt 86/2**

(45) Bekanntmachung des Hinweises auf die Patenterteilung: **09.03.88 Patentblatt 88/10**

(84) Benannte Vertragsstaaten: **AT BE CH DE FR GB IT LI NL**

(56) Entgegenhaltungen:
**EP - A - 0 087 388**
**DE - A - 3 129 429**

(73) Patentinhaber: **BAYER AG, Konzernverwaltung RP Patentabteilung, D-5090 Leverkusen 1 Bayerwerk (DE)**

(72) Erfinder: **Stetter, Jörg, Dr., Gellertweg 4, D-5600 Wuppertal 1 (DE)**
Erfinder: **Gehring, Reinhold, Dr., Dasnoeckel 49, D-5600 Wuppertal 1 (DE)**
Erfinder: **Lindig, Markus, Dr., Dahlienweg 16, D-4010 Hilden (DE)**
Erfinder: **Schallner, Otto, Dr., Noldeweg 22, D-4010 Monheim (DE)**
Erfinder: **Eue, Ludwig, Dr., Paul-Klee-Strasse 36, D-5090 Leverkusen 1 (DE)**
Erfinder: **Schmidt, Robert R., Dr., Im Waldwinkel 110, D-5060 Bergisch-Gladbach 2 (DE)**
Erfinder: **Santel, Hans-Joachim, Dr., Gerstenkamp 19, D-5000 Koeln 80 (DE)**
Erfinder: **Lürssen, Klaus, Dr., August-Kierspel-Strasse 151, D-5060 Bergisch-Gladbach 2 (DE)**

EP 0 167 028 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue 5-Amino-4-heterocyclyl-1-phenylpyrazole, mehrere Verfahren zu ihrer Herstellung und ihre Verwendung als Herbizide und Pflanzenwachstumsregulatoren.

Es ist bereits bekannt, dass bestimmte 5-Amino-1-phenylpyrazole, wie beispielsweise das 5-Amino-4-ethoxycarbonyl-1-(2,4,6-trichlorophenyl)-pyrazol, herbizide Eigenschaften besitzen (vergl. z.B. DE-OS 3 129 429).

Die Wirksamkeit dieser vorbekannten Verbindungen ist jedoch, insbesondere bei niedrigen Aufwandmengen und -konzentrationen, nicht immer in allen Anwendungsbereichen völlig zufriedenstellend.

Es wurden neue 5-Amino-4-heterocyclyl-1-phenylpyrazole der allgemeinen Formel (I),

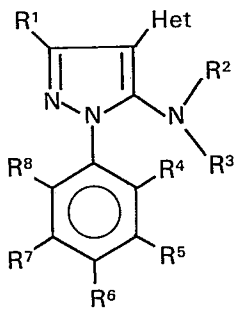

I

in welcher

$R^1$ für Wasserstoff oder für geradkettiges oder verzweigtes Alkyl mit 1 bis 8 Kohlenstoffatomen steht,

$R^2$ für Wasserstoff oder einen Rest $-\underset{X}{\underset{\|}{C}}-R^9$ steht,

$R^3$ unabhängig von $R^2$ für die gleichen Reste wie $R^2$ steht und zusätzlich für jeweils geradkettiges oder verzweigtes Alkyl, Alkenyl oder Alkinyl mit bis zu 4 Kohlenstoffatomen steht,

$R^4$, $R^5$, $R^6$, $R^7$ und $R^8$ unabhängig voneinander für Wasserstoff, Cyano, Nitro, Halogen oder für jeweils geradkettiges oder verzweigtes Alkyl, Alkoxy oder Alkoxycarbonyl mit jeweils bis zu 4 Kohlenstoffatomen, ausserdem für jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder für einen Rest $-S(O)_n-R^{10}$ stehen, wobei

$R^9$ für Wasserstoff, für jeweils geradkettiges oder verzweigtes Alkyl, Alkenyl, Alkinyl, Alkoxyalkyl, Alkylthioalkyl, Alkoxy, Alkylthio, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen, ausserdem für gegebenenfalls einfach oder mehrfach, gleich oder verschieden durch Halogen, niederes Alkyl oder niederes Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenoxyalkyl, Phenylthio oder Phenylamino steht, wobei als Phenylsubstituenten die bei $R^9$ genannten infrage kommen;

$R^{10}$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,

X für Sauerstoff oder Schwefel steht,

n für eine Zahl 0, 1 oder 2 steht und

Het für einen gegebenenfalls einfach oder mehrfach, gleich oder verschieden substituierten, gesättigten oder ungesättigten, fünf- oder sechsgliedrigen Heterocyclus steht, der ein bis drei gleiche oder verschiedene Heteroatome aus der Gruppe Stickstoff, Sauerstoff und Schwefel enthält und der über ein Kohlenstoff- oder ein Stickstoffatom verknüpft sein kann, wobei als Substituenten infrage kommen: Halogen, Nitro, sowie jeweils geradkettiges oder verzweigtes Alkyl, Alkoxy, Alkylthio oder Halogenalkyl mit jeweils bis zu 4 Kohlenstoffatomen und gegebenenfalls bis zu 9 gleichen oder verschiedenen Halogenatomen,

gefunden.

Weiterhin wurde gefunden, dass man die neuen 5-Amino-4-heterocyclyl-1-phenyl-pyrazole der allgemeinen Formel (I) erhält, wenn man

(a) Phenylhydrazine der Formel (II),

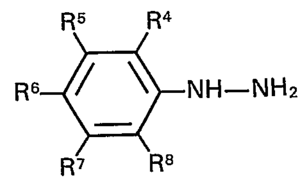

(II)

in welcher

$R^4$, $R^5$, $R^6$, $R^7$ und $R^8$ die oben angegebene Bedeutung haben,

mit Acrylnitril-Derivaten der Formel (III),

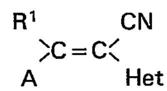

(III)

in welcher

$R^1$ und Het die oben angegebene Bedeutung haben und

A für Chlor, Brom, Methoxy, Ethoxy oder Dimethylamino steht,

entweder zunächst in einer 1. Stufe, gegebenenfalls in Gegenwart eines Verdünnungsmittels sowie gegebenenfalls in Gegenwart eines Reaktionshilfsmittels, umsetzt zu den Phenylhydrazin-Derivaten der Formel (IV),

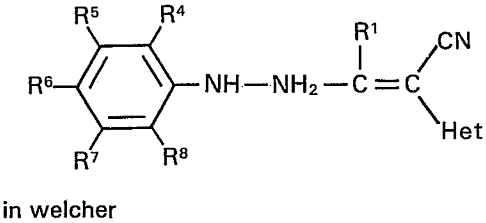

(IV)

in welcher

$R^1$, $R^4$, $R^5$, $R^6$, $R^7$, $R^8$ und Het die oben angegebene Bedeutung haben,

und diese in einer 2. Stufe, gegebenenfalls in Gegenwart eines Verdünnungsmittels und gegebenenfalls in Gegenwart eines sauren Katalysators, cyclisiert oder direkt in einem Reaktionsschritt ohne Isolierung der Zwischenstufe der Formel (IV), gegebenenfalls in Gegenwart eines Verdünnungsmittels, cyclisiert zu den 5-Amino-pyrazolen der Formel (Ia),

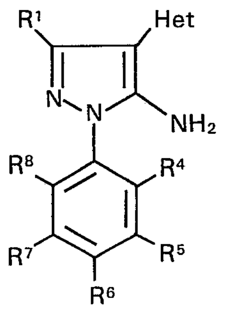

(Ia)

in welcher

$R^1$, $R^4$, $R^5$, $R^6$, $R^7$, $R^8$ und Het die oben angegebene Bedeutung haben,

oder wenn man

(b) die nach Verfahren (a) erhältlichen 5-Aminopyrazole der Formel (Ia)

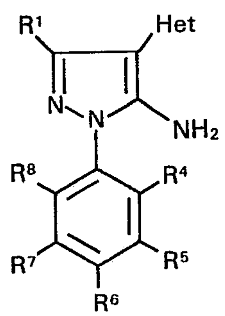

(Ia)

in welcher

$R^1$, $R^4$, $R^5$, $R^6$, $R^7$, $R^8$ und Het die oben angegebene Bedeutung haben,

in allgemein üblicher Weise mit Acylierungsmitteln oder Alkylierungsmitteln der Formel (V)

$$R^{11} - A' \qquad (V)$$

in welcher

$R^{11}$ für jeweils geradkettiges oder verzweigtes Alkyl, Alkenyl oder Alkinyl mit bis zu 4 Kohlenstoffatomen oder einen Rest $-\overset{\overset{\displaystyle X}{\|}}{C}-R^9$ steht, wobei X und $R^9$ die oben angegebene Bedeutung haben und

A' für Chlor, Brom, Iod, p-Toluolsulfonyloxy, Alkoxysulfonyloxy oder Acyloxy steht,

oder mit Iso(thio)cyanaten der Formel (VI),

$$R^{12} - N = C = X \qquad (VI)$$

in welcher

$R^{12}$ für geradkettiges oder verzweigtes Alkyl mit bis zu 4 Kohlenstoffatomen, oder für gegebenenfalls ein- bis dreifach, gleich oder verschieden substituiertes Phenyl steht, wobei als Substituenten infrage kommen: Halogen, jeweils geradkettiges oder verzweigtes Alkyl, Alkoxy oder Halogenalkyl mit jeweils bis zu 4 Kohlenstoffatomen und im Fall des Halogenalkyl mit 1 bis zu 9 gleichen oder verschiedenen Halogenatomen, und

X die oben angegebene Bedeutung hat,

gegebenenfalls in Gegenwart eines Verdünnungsmittels und gegebenenfalls in Gegenwart eines Säurebindemittels, umsetzt zu den am Stickstoff alkylierten bzw. acylierten 5-Amino-pyrazolen der Formel (Ib),

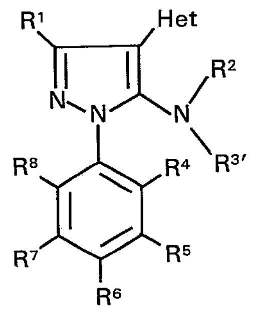

(Ib)

in welcher

$R^1$, $R^2$, $R^4$, $R^5$, $R^6$, $R^7$, $R^8$ und Het die oben angegebene Bedeutung haben und

$R^{3'}$ für die gleichen Reste, wie das oben angegebene $R^3$ mit Ausnahme des Wasserstoffrestes steht.

Schliesslich wurde gefunden, dass die neuen 5-Amino-4-heterocyclyl-1-phenylpyrazole der Formel (I) herbizide Eigenschaften, insbesondere auch selektive herbizide und pflanzenwachstumsregulatorische Eigenschaften besitzen.

Überraschenderweise zeigen die erfindungsgemässen 5-Amino-4-heterocyclyl-1-phenylpyrazole der Formel (I) eine erheblich bessere herbizide Wirksamkeit als die aus dem Stand der Technik bekannten 5-Amino-1-phenylpyrazole, wie beispielsweise das 5-Amino-4-ethoxycarbonyl-1-(2,4,6-trichlorphenyl)-pyrazol, welches chemisch und wirkungsmässig naheliegende Verbindungen sind.

Bevorzugt sind diejenigen Verbindungen Formel (I), bei welchen

$R^1$ für Wasserstoff, Methyl, Ethyl, n- oder i-Propyl, n-, i-, s- oder t-Butyl steht,

$R^2$ für Wasserstoff oder einen Rest $-\overset{\overset{\displaystyle X}{\|}}{C}-R^9$ steht,

$R^3$ unabhängig von $R^2$ für die gleichen Reste wie $R^2$ steht und zusätzlich für Methyl, Ethyl, n- und i-Propyl, n-, i-, s- und t-Butyl sowie Allyl und Propargyl steht,

$R^4$, $R^5$, $R^6$, $R^7$ und $R^8$ unabhängig voneinander für Wasserstoff, Cyano, Nitro, Fluor, Chlor, Brom, Jod, Methyl, Ethyl, n- und i-Propyl, n-, i-, s- und t-Butyl, für Methoxy, Ethoxy, Methoxycarbonyl, Ethoxycarbonyl, für Trifluormethyl, Trichlormethyl, Dichlorfluormethyl, Difluorchlormethyl,

Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, für Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest $-S(O)_n-R^{10}$ stehen, wobei

$R^9$ für Wasserstoff, Methyl, Ethyl, n- und i-Propyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Jodmethyl, Brommethyl, Dichlormethyl, 1-Chlorethyl, 2-Chlorethyl, 3-Chlorpropyl, 2-Bromethyl, Heptafluor-n-propyl, für jeweils gegebenenfalls ein- bis vierfach, gleich oder verschieden durch Fluor, Chlor, Brom, Methyl oder Trifluormethyl substituiertes Cyclopropyl, Cyclopentyl oder Cyclohexyl, sowie für jeweils gegebenenfalls ein-bis dreifach, gleich oder verschieden durch Methyl, Methoxy, Chlor oder Trifluormethyl substituiertes Phenyl, Phenoxy, Phenoxymethyl, Phenylthio oder Phenylamino steht,

$R^{10}$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht,

X für Sauerstoff oder Schwefel steht,

n für eine Zahl 0, 1 oder 2 steht und

Het für einen gegebenenfalls ein- bis dreifach, gleich oder verschieden durch Fluor, Chlor, Brom, Nitro, Methyl, Ethyl, n- und i-Propyl, Methoxy, Ethoxy, n- und i-Propoxy, Methylthio, Ethylthio, n- und i-Propylthio oder Trifluormethyl substituierten Heterocyclus der Formel

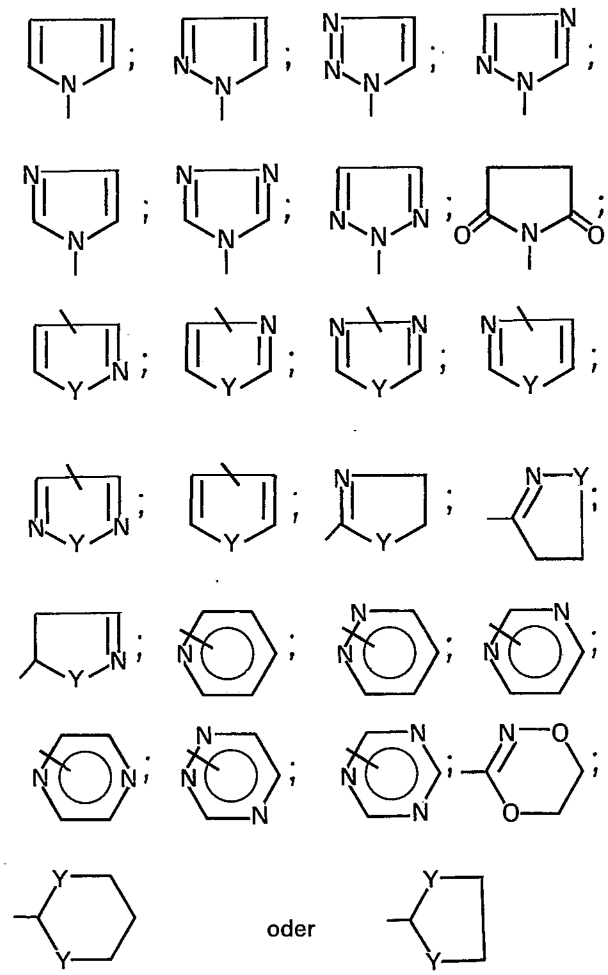

oder

steht, wobei

Y jeweils für Sauerstoff oder Schwefel oder einen N-Alkyl-Rest mit bis zu 4 Kohlenstoffatomen steht.

Im einzelnen seien ausser den bei den Herstellungsbeispielen genannten Verbindungen die folgenden 5-Amino-4-heterocyclyl-1-phenylpyrazole der allgemeinen Formel (I) genannt:

Tabelle 1

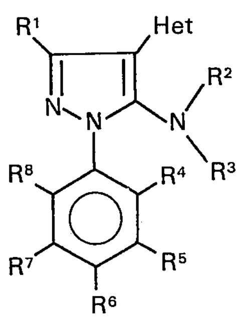

(I)

| $R^1$ | $R^2$ | $R^3$ | $R^4$ | $R^5$ | $R^6$ | $R^7$ | $R^8$ | Het |
|---|---|---|---|---|---|---|---|---|
| H | H | H | Cl | H | Cl | H | Cl | 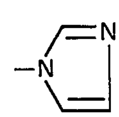 |

Tabelle 1 (Fortsetzung)

| $R^1$ | $R^2$ | $R^3$ | $R^4$ | $R^5$ | $R^6$ | $R^7$ | $R^8$ | Het |
|---|---|---|---|---|---|---|---|---|
| H | H | $-\overset{O}{\overset{\|}{C}}-CH_3$ | Cl | H | Cl | H | Cl | 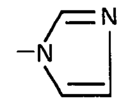 |
| H | H | $-\overset{O}{\overset{\|}{C}}-C_2H_5$ | Cl | H | Cl | H | Cl | 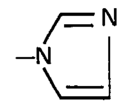 |
| H | H | H | Cl | H | $CF_3$ | H | Cl | 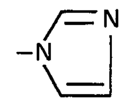 |
| H | H | $-\overset{O}{\overset{\|}{C}}-CH_3$ | Cl | H | $CF_3$ | H | Cl | 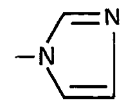 |
| H | H | H | Cl | H | $OCF_3$ | H | H | 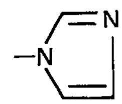 |
| H | H | $-\overset{O}{\overset{\|}{C}}-C_2H_5$ | Cl | H | $OCF_3$ | H | H | 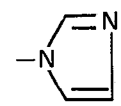 |
| H | H | H | Cl | H | $OCF_3$ | H | Cl | 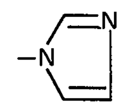 |
| H | H | $-\overset{O}{\overset{\|}{C}}-CH_3$ | Cl | H | $OCF_3$ | H | Cl | 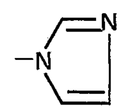 |
| H | H | $-\overset{O}{\overset{\|}{C}}-C_2H_5$ | Cl | H | $OCF_3$ | H | Cl | 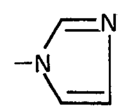 |
| H | H | H | Cl | H | $SCF_3$ | H | Cl | 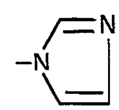 |
| H | H | $-\overset{O}{\overset{\|}{C}}-C_2H_5$ | Cl | H | $SCF_3$ | H | Cl | 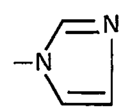 |
| H | H | H | Cl | H | $SO_2CF_3$ | H | Cl | 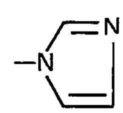 |
| H | H | $-\overset{O}{\overset{\|}{C}}-CH_3$ | Cl | H | $SO_2CF_3$ | H | Cl | 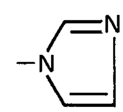 |
| H | H | $-\overset{O}{\overset{\|}{C}}-C_2H_5$ | Cl | H | $SO_2CF_3$ | H | Cl | 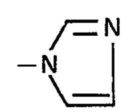 |
| H | H | H | Cl | H | Cl | H | Cl | 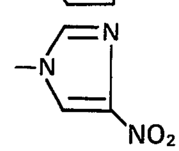 |

Tabelle 1 (Fortsetzung)

| $R^1$ | $R^2$ | $R^3$ | $R^4$ | $R^5$ | $R^6$ | $R^7$ | $R^8$ | Het |
|---|---|---|---|---|---|---|---|---|
| H | H | $-\overset{O}{\overset{\|}{C}}-C_2H_5$ | Cl | H | Cl | H | Cl | 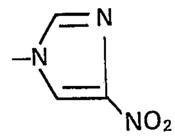 |
| H | H | H | Cl | H | $CF_3$ | H | Cl | 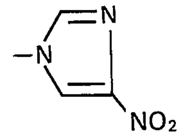 |
| H | H | $-\overset{O}{\overset{\|}{C}}-C_2H_5$ | Cl | H | $CF_3$ | H | Cl | 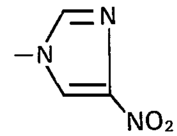 |
| H | H | H | Cl | H | $OCF_3$ | H | H | 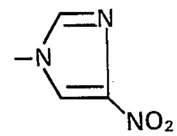 |
| H | H | $-\overset{O}{\overset{\|}{C}}-C_2H_5$ | Cl | H | $OCF_3$ | H | H | 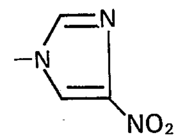 |
| H | H | H | Cl | H | Cl | H | Cl | 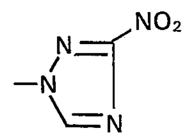 |
| H | H | $-\overset{O}{\overset{\|}{C}}-C_2H_5$ | Cl | H | Cl | H | Cl | 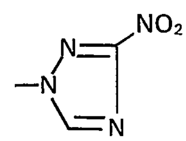 |
| H | H | H | Cl | H | $CF_3$ | H | Cl | 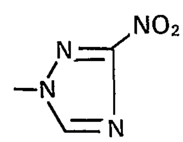 |
| H | H | $-\overset{O}{\overset{\|}{C}}-C_2H_5$ | Cl | H | $CF_3$ | H | Cl | 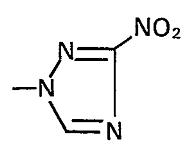 |
| H | H | H | Cl | H | $OCF_3$ | H | H | 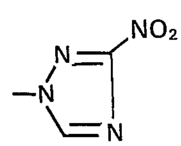 |

Tabelle 1 (Fortsetzung)

| $R^1$ | $R^2$ | $R^3$ | $R^4$ | $R^5$ | $R^6$ | $R^7$ | $R^8$ | Het |
|---|---|---|---|---|---|---|---|---|
| H | H | $-\overset{O}{\overset{\|}{C}}-C_2H_5$ | Cl | H | $OCF_3$ | H | H | 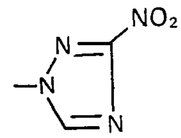 |
| H | H | H | Cl | H | Cl | H | Cl | 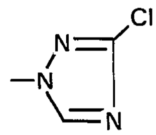 |
| H | H | $-\overset{O}{\overset{\|}{C}}-C_2H_5$ | Cl | H | CL | H | Cl | 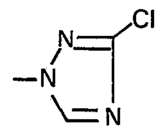 |
| H | H | H | Cl | H | $CF_3$ | H | Cl | 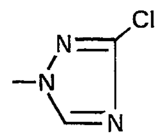 |
| H | H | $-\overset{O}{\overset{\|}{C}}-C_2H_5$ | Cl | H | $CF_3$ | H | Cl | 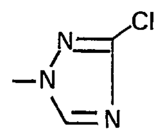 |
| H | H | H | Cl | H | Cl | H | Cl | 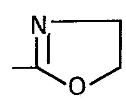 |
| H | H | $-\overset{O}{\overset{\|}{C}}-CH_3$ | Cl | H | Cl | H | Cl | 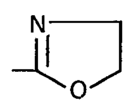 |
| H | H | H | Cl | H | $CF_3$ | H | Cl | 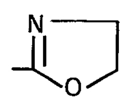 |
| H | H | $-\overset{O}{\overset{\|}{C}}-C_2H_5$ | Cl | H | $CF_3$ | H | Cl | 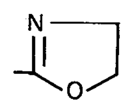 |
| H | H | H | Cl | H | $OCF_3$ | H | Cl | 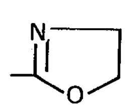 |
| H | H | $-\overset{O}{\overset{\|}{C}}-C_2H_5$ | Cl | H | $OCF_3$ | H | Cl | 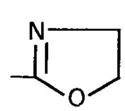 |
| H | H | H | Cl | H | $OCF_3$ | H | H | 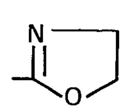 |
| H | H | $-\overset{O}{\overset{\|}{C}}-CH_3$ | Cl | H | $OCF_3$ | H | H | 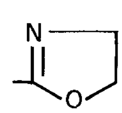 |

Tabelle 1 (Fortsetzung)

| $R^1$ | $R^2$ | $R^3$ | $R^4$ | $R^5$ | $R^6$ | $R^7$ | $R^8$ | Het |
|---|---|---|---|---|---|---|---|---|
| H | H | H | Cl | H | $CF_3$ | H | Cl | 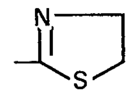 |
| H | H | $-\overset{O}{\overset{\parallel}{C}}-CH_3$ | Cl | H | $CF_3$ | H | Cl | 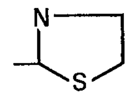 |
| H | H | H | Cl | H | Cl | H | Cl | 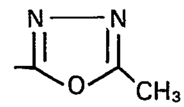 |
| H | H | $-\overset{O}{\overset{\parallel}{C}}-C_2H_5$ | Cl | H | Cl | H | Cl | 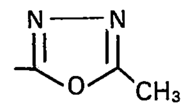 |
| H | H | H | Cl | H | $CF_3$ | H | Cl | 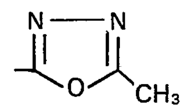 |
| H | H | $-\overset{O}{\overset{\parallel}{C}}-CH_3$ | Cl | H | $CF_3$ | H | Cl | 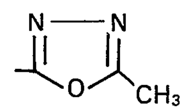 |
| H | H | H | Cl | H | $OCF_3$ | H | H | 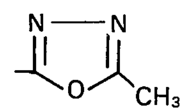 |
| H | H | H | Cl | H | $SO_2CF_3$ | H | Cl | 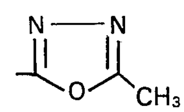 |
| H | H | $-\overset{O}{\overset{\parallel}{C}}-CH_3$ | Cl | H | $SO_2CF_3$ | H | Cl | 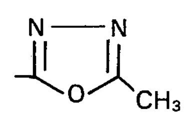 |
| H | H | H | Cl | H | Cl | H | Cl | 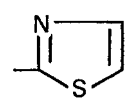 |
| H | H | $-\overset{O}{\overset{\parallel}{C}}-CH_3$ | Cl | H | Cl | H | Cl | 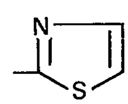 |

Tabelle 1 (Fortsetzung)

| $R^1$ | $R^2$ | $R^3$ | $R^4$ | $R^5$ | $R^6$ | $R^7$ | $R^8$ | Het |
|---|---|---|---|---|---|---|---|---|
| H | H | 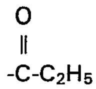 | Cl | H | Cl | H | Cl | 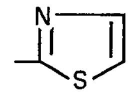 |
| H | H | H | Cl | H | $CF_3$ | H | Cl | 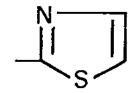 |
| H | H | 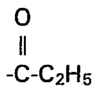 | Cl | H | $CF_3$ | H | Cl | 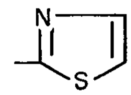 |
| H | H | H | Cl | H | $OCF_3$ | H | H | 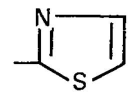 |
| H | H | H | Cl | H | $SCF_3$ | H | H | 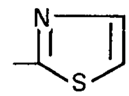 |
| H | H | H | Cl | H | $SO_2CF_3$ | H | H | 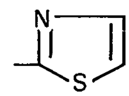 |
| H | H | $CH_3$ | Cl | H | Cl | H | Cl | 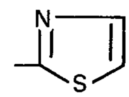 |
| H | H | $C_2H_5$ | Cl | H | Cl | H | Cl | 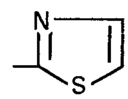 |
| H | H | 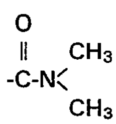 | Cl | H | Cl | H | Cl | 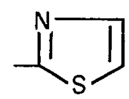 |
| H | H | 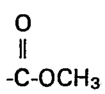 | Cl | H | Cl | H | Cl | 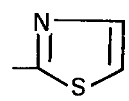 |
| H | H | 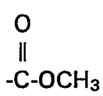 | Cl | H | $CF_3$ | H | Cl | 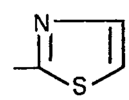 |
| H | H | 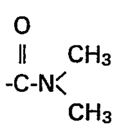 | Cl | H | $CF_3$ | H | Cl | 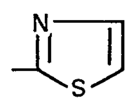 |
| H | H | 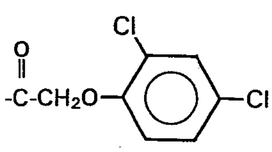 | Cl | H | CL | H | Cl | 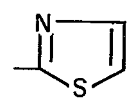 |
| H | H | 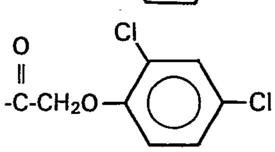 | Cl | H | Cl | H | Cl | 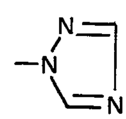 |

Tabelle 1 (Fortsetzung)

| $R^1$ | $R^2$ | $R^3$ | $R^4$ | $R^5$ | $R^6$ | $R^7$ | $R^8$ | Het |
|---|---|---|---|---|---|---|---|---|
| H | H | 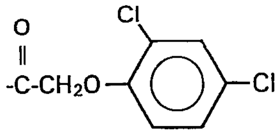 | Cl | H | Cl | H | Cl | 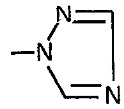 |
| H | H | 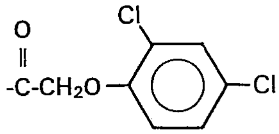 | Cl | H | $OCF_3$ | H | H | 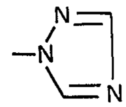 |
| H | H | 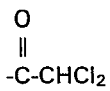 | Cl | H | Cl | H | Cl | 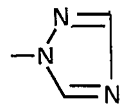 |
| H | H | 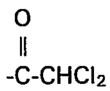 | Cl | H | $CF_3$ | H | Cl | 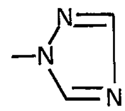 |
| H | H | 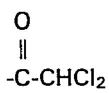 | Cl | H | $OCF_3$ | H | H | 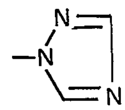 |
| H | H | 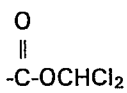 | Cl | H | $OCF_3$ | H | Cl | 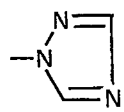 |
| H | H | 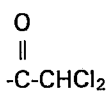 | Cl | H | $SO_2CF_3$ | H | Cl | 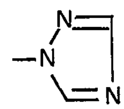 |
| H | H | H | Cl | H | Cl | H | Cl | 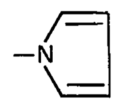 |
| H | H | 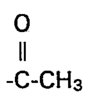 | Cl | H | Cl | H | Cl | 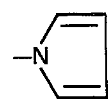 |
| H | H | 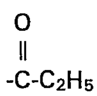 | Cl | H | Cl | H | Cl | 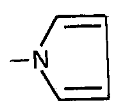 |
| H | H | H | Cl | H | $CF_3$ | H | Cl | 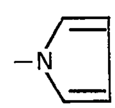 |
| H | H | 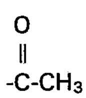 | Cl | H | $CF_3$ | H | Cl | 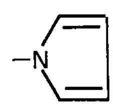 |
| H | H | 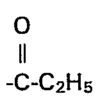 | Cl | H | $CF_3$ | H | Cl | 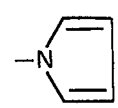 |
| H | H | H | Cl | H | $OCF_3$ | H | H | 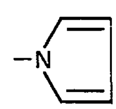 |

Tabelle 1 (Fortsetzung)

| $R^1$ | $R^2$ | $R^3$ | $R^4$ | $R^5$ | $R^6$ | $R^7$ | $R^8$ | Het |
|---|---|---|---|---|---|---|---|---|
| H | H | H | Cl | H | $OCF_3$ | H | Cl | 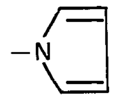 |
| H | H | H | Cl | H | $SO_2CF_3$ | H | Cl | 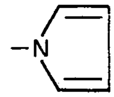 |
| H | H | H | Cl | H | Cl | H | Cl | 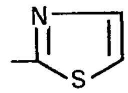 |
| H | H | H | Cl | H | $CF_3$ | H | Cl | 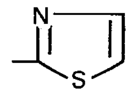 |
| H | H | O<br>‖<br>$-C-C_2H_5$ | Cl | H | Cl | H | Cl | 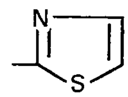 |
| H | H | O<br>‖<br>$-C-C_2H_5$ | Cl | H | $CF_3$ | H | Cl | 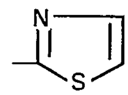 |
| H | H | H | Cl | H | Cl | H | Cl | 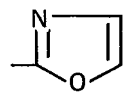 |
| H | H | H | Cl | H | $CF_3$ | H | Cl | 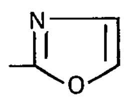 |
| H | H | H | Cl | H | $OCF_3$ | H | Cl | 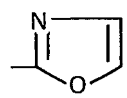 |
| H | H | O<br>‖<br>$-C-C_2H_5$ | Cl | H | $CF_3$ | H | Cl | 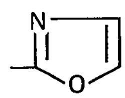 |
| H | H | H | Cl | H | Cl | H | Cl | 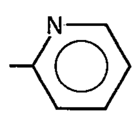 |
| H | H | H | Cl | H | $CF_3$ | H | Cl | 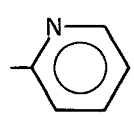 |
| H | H | H | Cl | H | $OCF_3$ | H | Cl | 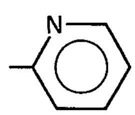 |
| H | H | O<br>‖<br>$-C-C_2H_5$ | Cl | H | Cl | H | Cl | 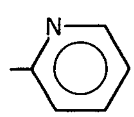 |

Tabelle 1 (Fortsetzung)

| $R^1$ | $R^2$ | $R^3$ | $R^4$ | $R^5$ | $R^6$ | $R^7$ | $R^8$ | Het |
|---|---|---|---|---|---|---|---|---|
| H | H | $-\overset{O}{\overset{\|}{C}}-C_2H_5$ | Cl | H | $CF_3$ | H | Cl | 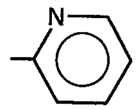 |
| H | H | H | Cl | H | Cl | H | Cl | 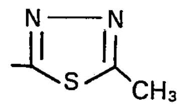 |
| H | H | H | Cl | H | $CF_3$ | H | Cl | 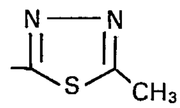 |
| H | H | H | Cl | H | $SO_2CH_3$ | H | Cl | 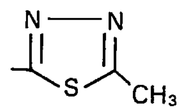 |
| H | H | $-\overset{O}{\overset{\|}{C}}-C_2H_5$ | Cl | H | $CF_3$ | H | Cl | 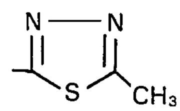 |
| H | H | H | Cl | H | Cl | H | Cl | 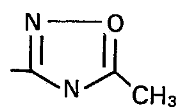 |
| H | H | H | Cl | H | $CF_3$ | H | Cl | 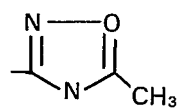 |
| H | H | H | Cl | H | Cl | H | Cl | 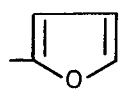 |
| H | H | H | Cl | H | $CF_3$ | H | Cl | 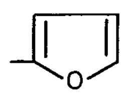 |
| H | H | $-\overset{O}{\overset{\|}{C}}-C_2H_5$ | Cl | H | $CF_3$ | H | Cl | 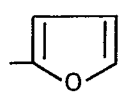 |
| H | H | $-\overset{O}{\overset{\|}{C}}-C_2H_5$ | Cl | H | Cl | H | Cl | 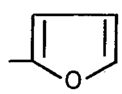 |
| H | H | H | Cl | H | Cl | H | Cl | 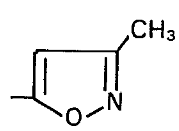 |
| H | H | H | Cl | H | $CF_3$ | H | Cl | 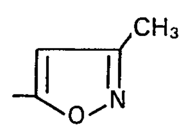 |

Tabelle 1 (Fortsetzung)

| $R^1$ | $R^2$ | $R^3$ | $R^4$ | $R^5$ | $R^6$ | $R^7$ | $R^8$ | Het |
|---|---|---|---|---|---|---|---|---|
| H | H | H | Cl | H | $OCF_3$ | H | Cl | 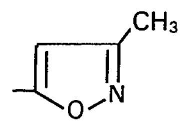 |
| H | H | H | Cl | H | $OCF_3$ | H | H | 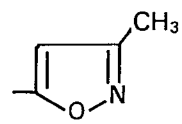 |
| H | H | H | Cl | H | Cl | H | Cl | 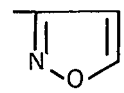 |
| H | H | H | Cl | H | $CF_3$ | H | Cl | 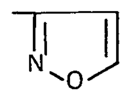 |
| H | H | H | Cl | H | $SO_2CF_3$ | H | Cl | 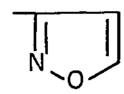 |
| H | H | H | Cl | H | $SCF_3$ | H | Cl | 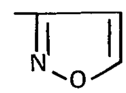 |
| H | H | H | Cl | H | Cl | H | Cl | 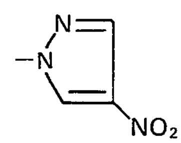 |
| H | H | H | Cl | H | $CF_3$ | H | Cl | 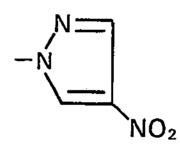 |
| H | H | $-\overset{O}{\overset{\|}{C}}-C_2H_5$ | Cl | H | $CF_3$ | H | Cl | 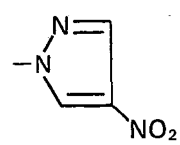 |
| H | H | H | Cl | H | Cl | H | Cl | 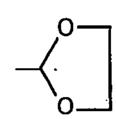 |
| H | H | $-\overset{O}{\overset{\|}{C}}-CH_3$ | Cl | H | Cl | H | Cl | 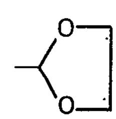 |
| H | H | $-\overset{O}{\overset{\|}{C}}-C_2H_5$ | Cl | H | Cl | H | Cl | 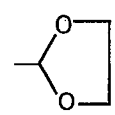 |
| H | H | H | Cl | H | $CF_3$ | H | Cl | 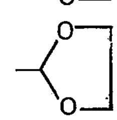 |

Tabelle 1 (Fortsetzung)

| $R^1$ | $R^2$ | $R^3$ | $R^4$ | $R^5$ | $R^6$ | $R^7$ | $R^8$ | Het |
|---|---|---|---|---|---|---|---|---|
| H | H | $\overset{O}{\overset{\|}{-C}}-CH_3$ | Cl | H | $CF_3$ | H | Cl | 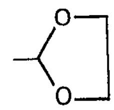 |
| H | H | $\overset{O}{\overset{\|}{-C}}-C_2H_5$ | Cl | H | $CF_3$ | H | Cl | 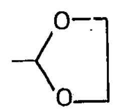 |
| H | H | H | Cl | H | $SO_2CF_3$ | H | Cl | 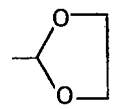 |
| H | H | $\overset{O}{\overset{\|}{-C}}-C_2H_5$ | Cl | H | $SO_2CF_3$ | H | Cl | 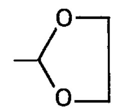 |
| H | H | H | Cl | H | $SCF_3$ | H | Cl | 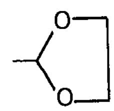 |
| H | H | H | Cl | H | $OCF_3$ | H | H | 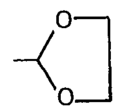 |
| H | H | H | Cl | H | $OCF_3$ | H | H | 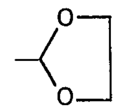 |

Verwendet man als Ausgangsstoffe beispielsweise 2,6-Dichlor-4-trifluormethylphenylhydrazin und 3-Dimethylamino-2-(1,2,4-triazol-1-yl)-acrylnitril-Hydrochlorid, so lässt sich der Reaktionsablauf des erfindungsgemässen Verfahrens (a) durch das folgende Formelschema darstellen:

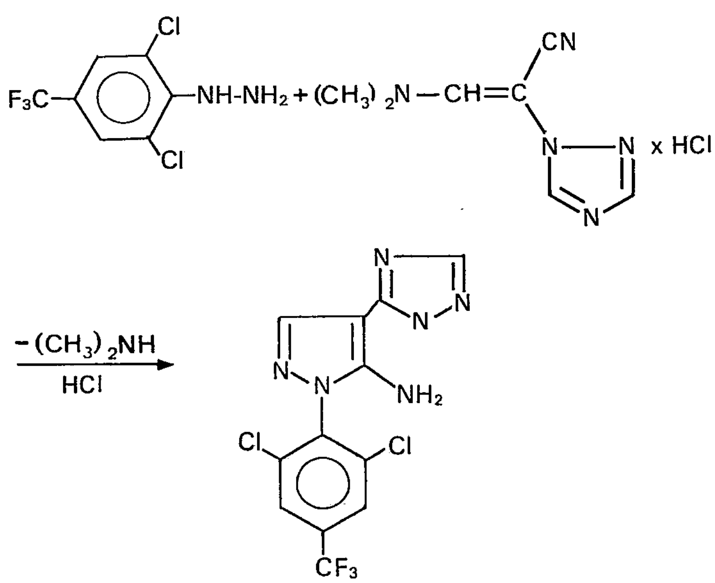

Verwendet man beispielsweise 1-(2,6-Dichlor-4-trifluormethylphenyl)-5-amino-4-(1,2,4-triazol-1-yl)-pyrazol und Propionylchlorid als Ausgangsstoffe, so lässt sich der Reaktionsablauf des erfindungsgemässen Verfahrens (b) durch das folgende Formelschema darstellen:

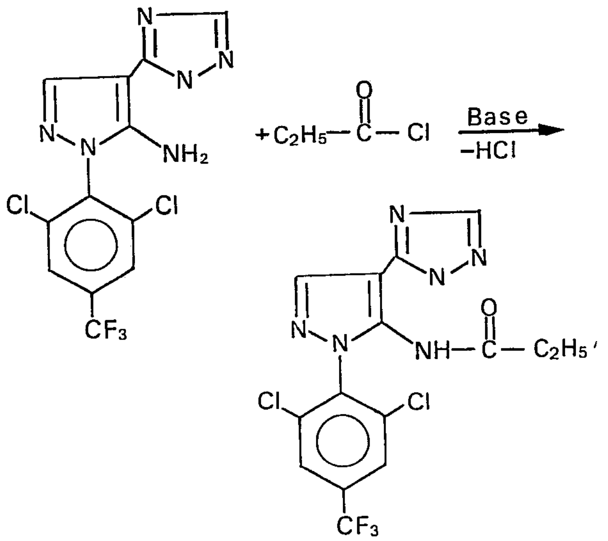


Die zur Durchführung des erfindungsgemässen Verfahrens (a) als Ausgangsstoffe benötigten Phenylhydrazine sind durch die Formel (II) allgemein definiert. In dieser Formel (II) stehen $R^4$, $R^5$, $R^6$, $R^7$ und $R^8$ vorzugsweise für diejenigen Substituenten, die bereits bei der Beschreibung der erfindungsgemässen Stoffe der Formel (I) als bevorzugt für diese Reste genannt wurden.

Die Phenylhydrazine der Formel (II) sind grösstenteils bekannt oder können nach bekannten Verfahren in einfacher, analoger Weise hergestellt werden (vgl.: z.B. Houben-Weyl 'Methoden der organischen Chemie', Band X, 2, S. 203, Thieme Verlag Stuttgart (1967), indem man beispielsweise die bekannten Aniline der Formel (VII),

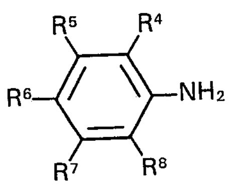
(VII)

in welcher

$R^4$, $R^5$, $R^6$, $R^7$ und $R^8$ die oben angegebene Bedeutung haben,

mit Natriumnitrit, In Gegenwart einer Säure, wie beispielsweise Schwefelsäure, und dann mit Zinn-(II)-chlorid, ebenfalls in Gegenwart einer Säure, wie beispielsweise Salzsäure, bei Temperaturen zwischen -20°C und +80°C umsetzt.

Die weiterhin zur Durchführung des Herstellungsverfahrens (a) als Ausgangsstoffe benötigten Acrylnitril-Derivate sind durch die Formel (III) allgemein definiert. In dieser Formel (III) stehen $R^1$ und Het vorzugsweise für diejenigen Reste, die schon bei der Beschreibung der erfindungsgemässen Stoffe der Formel (I) als bevorzugt für diese Substituenten genannt wurden. A steht für Chlor, Brom, Methoxy, Ethoxy oder Dimethylamino.

Die Acrylnitril-Derivate der Formel (III) sind noch nicht bekannt. Man erhält sie, wenn man Acetonitril-Derivate der Formel (VIII),

$$Het - CH_2 - CN \qquad (VIII)$$

in welcher

Het die oben angegebene Bedeutung hat,

entweder mit Orthoestern der Formel (IX),

$$R^1 - C \begin{cases} OR^{13} \\ OR^{13} \\ OR^{13} \end{cases} \qquad (IX)$$

in welcher

$R^1$ die oben angegebene Bedeutung hat und

$R^{13}$ für Methyl oder Ethyl steht,

oder mit Amidacetalen der Formel (X),

$$R^1 - C \begin{cases} OR^{14} \\ OR^{14} \\ A' \end{cases} \qquad (X)$$

in welcher

$R^1$ die oben angegebene Bedeutung hat,

$R^{14}$ für Methyl oder Ethyl steht und

A' für Dimethylamino steht,

gegebenenfalls in Gegenwart eines Verdünnungsmittels wie beispielsweise Toluol bei Temperaturen zwischen +80°C und +200°C umsetzt,

oder mit Estern der Formel (XI),

$$R^1 - \overset{\overset{O}{\|}}{C} - O - R^{15} \qquad (XI)$$

in welcher

$R^1$ die oben angegebene Bedeutung hat und

$R^{15}$ für Methyl oder Ethyl steht,

gegebenenfalls in Gegenwart eines Verdünnungsmittels, wie beispielsweise Methanol oder Ethanol, und gegebenenfalls in Gegenwart eines basischen Katalysators, wie beispielsweise Natriummethylat oder -ethylat, bei Temperaturen zwischen 0°C und 100°C umsetzt und die so erhältlichen Hydroxyverbindungen der Formel (IIIa),

$$\begin{matrix} Het \\ NC \end{matrix} \rangle C = C \langle \begin{matrix} R^1 \\ OH \end{matrix} \qquad (IIIa)$$

in welcher

$R^1$ und Het die oben angegebene Bedeutung haben,

in üblicher Weise mit Halogenierungsmitteln, wie Phosphorpentachlorid oder Thionylchlorid oder Phosphortribromid, bei Temperaturen zwischen 0°C und +100°C substituiert.

Die Aniline der Formel (VII) und die Acetonitril-Derivate der Formel (VIII) sind bekannt [vgl. z.B. B. Zh. org. Khim. 18, 463 (1982) s.a.C.A. 96, 181213x; DE-OS 3 129 429; Ber. dtsch. chem. Ges. 27, 3151 (1984)] oder lassen sich nach bekannten Verfahren in einfacher analoger Weise herstellen.

Die Orthoester der Formel (IX), die Amidacetale der Formel (X) und die Ester der Formel (XI) sind allgemein bekannte Verbindungen der organischen Chemie.

Die zur Durchführung des erfindungsgemässen Verfahrens (B) als Ausgangsstoffe benötigten 5-Amino-pyrazole sind durch die Formel (Ia) allgemein definiert. In dieser Formel (Ia) stehen, $R^1$, $R^4$, $R^5$, $R^6$, $R^7$, $R^8$ und Het vorzusweise für diejenigen Reste, die schon bei der Beschreibung der erfindungsgemäss verwendbaren Stoffe der Formel (I) als bevorzugt für diese Substituenten genannt wurden.

Die 5-Aminopyrazole der Formel (Ia) sind erfindungsgemässe Verbindungen und erhältlich nach Herstellungsverfahren (a).

Die Alkylierungs- und Acylierungsmittel der Formel (V) sind allgemein bekannte Verbindungen der organischen Chemie.

Die alternativ zur Durchführung des Herstellungsverfahrens (b) als Ausgangsstoffe verwendbaren Iso(thio)cyanate sind durch die Formel (VI) allgemein definiert. In dieser Formel steht X für Sauerstoff oder Schwefel und $R^{12}$ steht vorzugsweise für Methyl, Ethyl und gegebenenfalls ein- bis dreifach, gleich oder verschieden durch Fluor, Chlor, Methyl, Methoxy oder Trifluormethyl substituiertes Phenyl.

Die Iso(thio)cyanate der Formel (VI) sind ebenfalls allgemein bekannte Verbindungen der organischen Chemie.

Als Verdünnungsmittel zur Durchführung des Herstellungsverfahrens (a) kommen sowohl für die 1. als auch für die 2. Reaktionsstufe inerte organische Lösungsmittel infrage. Vorzugsweise verwendet man Alkohole, wie Methanol, Ethanol, Propanol, Butanol, Ethylenglykol oder Ethylenglykolmonomethyl- oder -ethylether.

Als Reaktionshilfsmittel zur Durchführung der 1. Stufe des Herstellungsverfahrens (a) kommen organische oder anorganische Säuren infrage. Vorzugsweise verwendet man Schwefelsäure oder Essigsäure, gegebenenfalls auch in Gegenwart einer Puffersubstanz, wie beispielsweise Natriumacetat.

Die Reaktionstemperaturen können bei der Durchführung der 1. Stufe des Herstellungsverfahrens (a) in gewissen Bereichen variiert werden. Im allgemeinen arbeitet man zwischen −30°C und +50°C, vorzugsweise zwischen −20°C und +20°C.

Als Katalysatoren zur Durchführung der 2. Stufe des Herstellungsverfahrens (a) kommen alle üblicherweise verwendbaren Säuren, wie beispielsweise Schwefelsäure oder Phosphorsäure infrage.

Die Reaktionstemperaturen können bei der Durchführung der 2. Stufe des Herstellungsverfahrens (a) ebenso wie bei der einstufigen Reaktionsführung in einem grösseren Bereich variiert werden. Im allgemeinen arbeitet man zwischen 0°C und +200°C, vorzugsweise zwischen +50°C und 150°C.

Zur Durchführung des Herstellungsverfahrens (a) setzt man sowohl bei der einstufigen als auch bei der zweistufigen Reaktionsführung pro Mol Phenylhydrazin der Formel (II) im allgemeinen 1,0 bis 3,0 Mol, vorzugsweise 1,0 bis 1,5 Mol an Acrylnitril-Derivat der Formel (III) und im Fall des zweistufigen Verfahrens gegebenenfalls in der 1. Stufe 1,0 bis 10,0 Mol an Reaktionshilfsmittel und gegebenenfalls in der 2. Stufe 1,0 bis 10,0 Mol an saurem Katalysator ein.

Die Aufarbeitung und Isolierung der Reaktionsprodukte erfolgt nach üblichen Verfahren, beispielsweise durch Entfernen des organischen Verdünnungsmittels, Ausfällen des Reaktionsproduktes in Wasser, Absaugen und Trocknen des so erhaltenen Produktes.

Zur Durchführung des Herstellungsverfahrens (b) kommen als Verdünnungsmittel ebenfalls inerte organische Lösungsmittel infrage. Vorzugsweise verwendet man aliphatische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Nitrile, wie Acetonitril oder Propionitril, Amide, wie Dimethylformamid, Dimethylacetamid, N-Methylpyrrolidon oder Hexamethylphosphorsäuretriamid. Verwendet man Acylierungsmittel oder Alkylierungsmittel der Formel (V) oder (VI) in flüssiger Form, so ist es auch möglich, diese in entsprechendem Überschuss als Verdünnungsmittel einzusetzen.

Als Säurebindemittel zur Durchführung des Herstellungsverfahrens (b) kommen alle üblicherweise verwendbaren anorganischen und organischen Basen infrage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (b) in einem grösseren Bereich variiert werden. Im allgemeinen arbeitet man zwischen −20°C und +150°C, vorzugsweise zwischen 0°C und +100°C.

Zur Durchführung des Herstellungsverfahrens (b) setzt man pro Mol 5-Amino-pyrazol der Formel (Ia) im allgemeinen 1,0 bis 20,0 Mol, vorzugsweise 1,0 bis 15,0 Mol an Acylierungs- oder Alkylierungsmittel der Formel (V) oder (VI) und gegebenenfalls 1,0 bis 3,0 Mol, vorzugsweise 1,0 bis 2,0 Mol an Säurebindemittel ein. Die Reaktionsdurchführung, Aufarbeitung und Isolierung der Reaktionsprodukte der Formel (Ib) erfolgt in allgemein üblicher Art und Weise.

Die erfindungsgemässen Wirkstoffe können als Defoliants, Dessicants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemässen Stoffe als totale oder selektive Herbizide wirken, hängt im wesentlichen von der angewendeten Menge ab.

Die erfindungsgemässen Wirkstoffe können z.B. bei den folgenden Pflanzen verwendet werden:

*Dikotyle Unkräuter der Gattungen:* Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

*Dikotyle Kulturen der Gattungen:* Gossypium, Glycina, Beta, Daucus, Phaseolus, Pisum, Solanum, Linum, Ipomoea, Vicia, Nicotiana, Lycopersicon, Arachis, Brassica, Lactuca, Cucumis, Cucurbita.

*Monokotyle Unkräuter der Gattungen:* Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

*Monokotyle Kulturen der Gattungen:* Oryza, Zea, Triticum, Hordeum, Avena, Secale, Sorghum, Panicum, Saccharum, Ananas, Asparagus, Allium.

Die Verwendung der erfindungsgemässen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassein sich die erfindungsgemässen Wirkstoffe mit besonders gutem Erfolg als selektive Unkrautbekämpfungsmittel von mono- und dikotylen Unkräutern, insbesondere in monokotylen Kulturen wie Mais oder Weizen, einsetzen.

Die erfindungsgemässen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, dass ein Wirkstoff auch mehrere verschiedenartige Wirkungen auf Pflanzen ausüben kann. Die Wirkungen der Stoffe hängen im wesentlichen ab von dem Zeitpunt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Strassenrändern, auf Flughäfen oder in Obstanlagen reduziert werden. Von bedeutung ist auch die Hemmung des Wuchses von krautigen und holzigen Pflanzen an Strassenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens («Lagerns») der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne dass die Gefahr besteht, dass das Getreide lagert.

Eine Hemmung des vegetativen Wachstums ermöglicht bei vielen Kulturpflanzen eine dichtere Anpflanzung, so dass Mehrerträge bezogen auf die Bodenfläche erzielt werden können. Ein Vorteil der so erzielten kleineren Pflanzen ist auch, dass die Kultur leichter bearbeitet und beerntet werden kann.

Eine Hemmung des vegetativen Wachstums der Pflanzen kann auch dadurch zu Ertragssteigerungen führen, dass die Nährstoffe und Assimilate in stärkerem Masse der Blüten- und Fruchtbildung zugute kommen als den vegetativen Pflanzenteilen.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, dadurch dass mehr Assimilate gebildet werden, so dass mehr oder grössere Früchte entstehen.

Ertragssteigerungen können in manchen Fällen durch einen Eingriff in den pflanzlichen Stoffwechsel erreicht werden, ohne dass sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Ausserdem lässt sich die Produktion oder der Abfluss von sekundären Pflanzeninhaltsstoffen positiv beeinflussen. Als Beispiel sei die Stimulierung des Latexflusses bei Gummibäumen genannt.

Unter dem Einfluss von Wachstumsregulatoren kann es zur Ausbildung parthenokarper Früchte kommen. Ferner kann das Geschlecht der Blüten beeinflusst werden. Auch kann eine Sterilität des Pollens erzeugt werden, was bei der Züchtung und Herstellung von Hybridsaatgut eine grosse Bedeutung hat.

Durch den Einsatz von Wachstumsregulatoren lässt sich die Verzweigung der Pflanzen steuern. Einerseits kann durch Brechen der Apikaldominanz

die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. grosses Interesse im Tabakanbau oder bei der Anpflanzung von Tomaten.

Unter dem Einfluss von Wachstumsregulatoren kann der Blattbestand der Pflanzen so gesteuert werden, dass ein Entblättern der Pflanzen zu einem gewünschten Zeitpunkt erreicht wird. Eine derartige Entlaubung spielt bei der mechanischen Beerntung der Baumwolle eine grosse Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso lässt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Masse gefördert werden («Ausdünnung»), um die Alternanz zu brechen. Unter Alternanz versteht man die Eigenart einiger Obstarten, endogen bedingt von Jahr zu Jahr sehr unterschiedliche Erträge zu bringen, Schliesslich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren lässt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, dass z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schliesslich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweisshydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die erfindungsgemässen Wirkstoffe können bei

der Anwendung als Herbizide als solche oder in ihren Formulierungen auch in Mischung mit bekannten Herbiziden zur Unkrautbekämfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfrass, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher weise, z.B. durch Giessen, Spritzen, Sprühen, Streuen.

Die erfindungsgemässen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann bei der Anwendung als Herbizid in einem grösseren Bereich schwanken. Sie hängt im wesentlichen von der Art des gewünschten Effektes ab. Im allgemeinen liegen die Aufwandmengen zwsichen 0,01 und 10 kg Wirkstoff pro Hektar Bodenfläche, vorzugsweise zwischen 0,05 und 5,0 kg pro ha.

Bei der Anwendung als Herbizide und Wachstumsregulatoren enthalten die Formulierungen im allgemeinen zwischen 0,1 und 95 Gewichtsprozent Wirkstoff, vorzugsweise zwischen 0,5 und 90%.

Die erfindungsgemässen Wirkstoffe können bei der Anwendung als Wachstumsregulatoren in den Formulierungen ebenfalls in Mischung mit anderen bekannten Wirkstoffen vorliegen, wie Fungizide, Insektizide, Akarizide und Herbizide, sowie in Mischung mit Düngemitteln und anderen Wachstumsregulatoren.

Die Wirkstoffe können dabei ebenfalls als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Giessen, Verspritzen, Versprühen, Verstreuen, Verstäuben, Bestreichen usw. Es ist ferner möglich, die Wirkstoffe nach dem Ultra-Low-Volumen-Verfahren auszubringen oder die Wirkstoffzubereitung oder den Wirkstoff selbst in den Boden zu injizieren. Es kann auch das Saatgut der Pflanzen behandelt werden.

Bei der Anwendung als Wachstumsregulatoren können die Aufwandmengen ebenfalls in einem grössere Bereich variiert werden. Im allgemeinen verwendet man pro Hektar Bodenfläche 0,01 bis 50 kg, bevozugt 0,05 bis 10 kg an Wirkstoff.

Für die Anwendungszeit gilt, dass die Anwendung der Wachstumsregulatoren in einem bevorzugten Zeitraum vorgenommen wird, dessen genaue Abgrenzung sich nach den klimatischen und vegetativen Gegebenheiten richtet.

Ausserdem zeigen die erfindungsgemässen Wirkstoffe auch fungizide und bakterizide Eigenschaften und können in geeigneten Aufwandmengen auch als protektive oder systemische Fungizide beispielsweise gegen den Erreger der Reisfleckenkrankheit (Pyricularia oryzae) oder gegen Schorfpilze eingesetzt werden.

Die nachfolgenden Beispiele dienen zur weiteren Erläuterung der Erfindung.

*Herstellungsbeispiele*

*Beispiel 1*

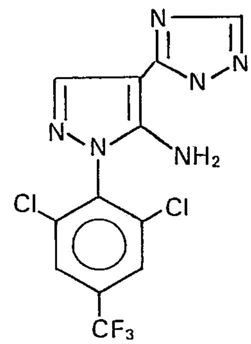

(1)

(Verfahren a)

19 g (0,075 Mol) 2,6-Dichlor-4-trifluormethylphenylhydrazin und 16 g (0,075 MoL) 3-Dimethylamino-2-(1,2,4-triazol-1-yl)-acrylnitril-Hydrochlorid in 200 ml Ethanol werden unter Rühren für 5 Stunden auf 75°C bis 78°C erhitzt. Zur Aufarbeitung wird das Lösungsmittel abdestilliert, der Rückstand mit Wasser angerieben und auf Ton getrocknet. Man erhält 26 g (96% der Theorie) an 5-Amino-1-(2,6-dichlor-4-trifluormethylphenyl)-4-(1,2,4-triazol-1-yl)-pyrazol vom Schmelzpunkt 170 - 174°C.

*Herstellung der Ausgangsverbindung:*

a) 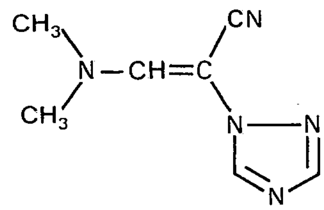 x HCl

108 g (1 Mol) 1,2,4-Triazol-1-yl-acetonitril und 160 g (1,35 Mol) N,N-Dimethylformamid-dimethylacetal werden 4 Stunden unter Rückfluss erhitzt; das entstandene Methanol wird abdestilliert bis die Innentemperatur auf 120°C angestiegen ist; der erhaltene Rückstand wird in 400 ml Ethanol gelöst und mit 400 ml einer 20%igen ethanolischen Chlorwasserstofflösung versetzt. Der kristalline Niederschlag wird abgesaugt, mit wenig Ethanol nachgewaschen und auf Ton getrocknet. Man erhält 163 g (82% der Theorie) an 3-Dimethylamino-2-(1,2,4-triazol-1-yl)-acrylnitril-Hydrochlorid vom Schmelzpunkt 200 bis 204°C.

(b) 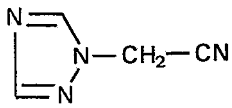

Zu einer Mischung aus 69 g (1 Mol) 1,2,4-Triazol, 150 g (1,08 Mol) gemahlenem Kaliumcarbonat und 500 ml Acetonitril gibt man tropfenweise unter Rühren bei 65-70°C während 30 Minuten 76 g (1 Mol) Chloracetonitril, rührt nach beendeter Zugabe weitere 3 Stunden bei 70°C, filtriert aus der erhaltenen Reaktionsmischung den unlöslichen Niederschlag ab, engt das Filtrat im Vakuum ein und destilliert den Rückstand im Hochvakuum. Man erhält 75 g (69% der Theorie) an 1,2,4-Triazol-1-yl-acetonitril vom Siedepunkt 85°C, bei 0,01 mbar, welches beim Erkalten erstarrt und einen Schmelzpunkt von 55°C bis 58°C besitzt.

*Beispiel 2*

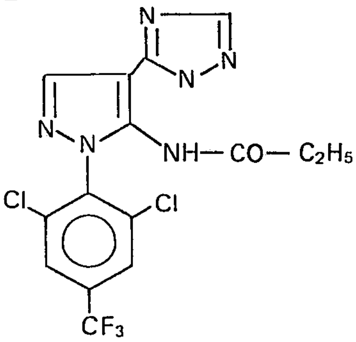

(2)

Zu einer Lösung von 7,3 g (0,02 Mol) 5-Amino-1-(2,6-dichlor-4-trifluormethylphenyl)-4-(1,2,4-trizol-1-yl)-pyrazol und 2 g (0,025 Mol) Pyridin in 60 ml Acetonitril tropft man unter Rühren 3,5 g (0,037 Mol) Propionylchlorid und rührt nach beendeter Zugabe weitere 12 bis 15 Stunden bei Raumtemperatur. Zur Aufarbeitung giesst man den Reaktionsansatz in Wasser, extrahiert mit Dichlormethan, wäscht die organische Phase mit Wasser, trocknet über Natriumsulfat und enfernt das Lösungsmittel im Vakuum. Der Rückstand wird in 100 ml Ethanol gelöst, mit 10 ml wässrigem Ammoniak versetzt, für 15 Minuten auf 50°C erhitzt, im Vakuum eingeengt, mit Wasser angerieben und auf Ton getrocknet. Man erhält 6,8 g (81% der Theorie) an 1-(2,6-Dichlor-4-trifluormethyl-phenyl)-5-propionylamino-4-(1,2,4-triazol-1-yl)-pyrazol vom Schmelzpunkt 167°C bis 169°C.

In entsprechender Weise und gemäss den allgemeinen Herstellungsangaben erhält man die folgenden Verbindungen der allgemeinen Formel (I).

Tabelle 2

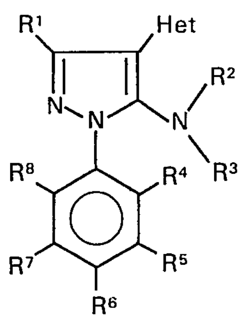

(I)

| Beispiel Nr. | $R^1$ | $R^2$ | $R^3$ | 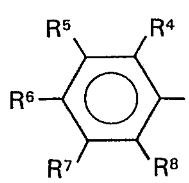 | Het | physikalische Eigenschaften |
|---|---|---|---|---|---|---|
| 3 | H | H | H | 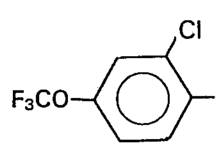 | 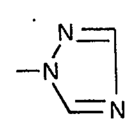 | Fp. 116-118°C |

Tabelle 2 (Fortsetzung)

| Beispiel Nr. | $R^1$ | $R^2$ | $R^3$ | 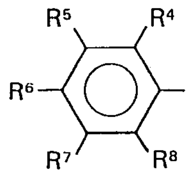 | Het | physikalische Eigenschaften |
|---|---|---|---|---|---|---|
| 4 | H | H | $C_2H_5CO$- | 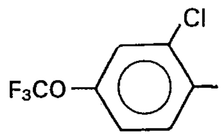 | 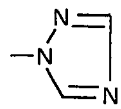 | Fp. 108-111°C |
| 5 | H | H | H | 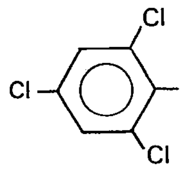 | 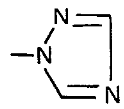 | Fp. 165-167°C |
| 6 | H | H | $C_2H_5CO$- | 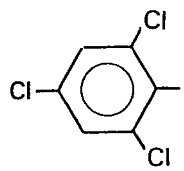 | 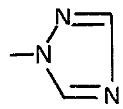 | Fp. 205-210°C |
| 7 | H | H | $CH_3O$-$CH_2$<br>CO- | 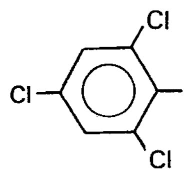 | 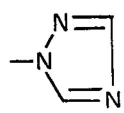 | Fp. 140-145°C |
| 8 | H | H | $CH_3CO$- | 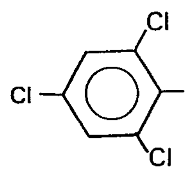 | 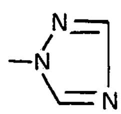 | Fp. 198°C |
| 9 | H | H | H | 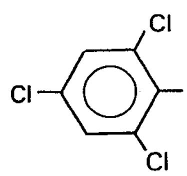 | 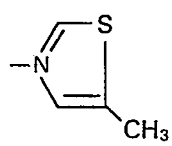 | Fp. 141-143°C |
| 10 | H | H | H | 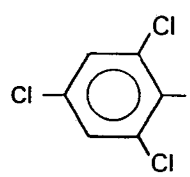 | 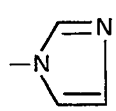 | Fp. 121-124°C |
| 11 | H | H | O<br>‖<br>$C_2H_5$-C- | 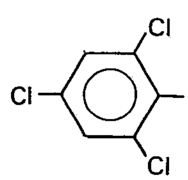 | 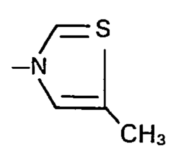 | |

Tabelle 2 (Fortsetzung)

| Bei-spiel Nr. | $R^1$ | $R^2$ | $R^3$ | 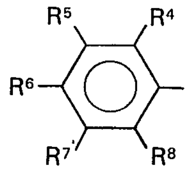 | Het | physikalische Eigenschaften |
|---|---|---|---|---|---|---|
| 12 | H | H | $C_2H_5\text{-}\overset{\overset{O}{\|}}{C}\text{-}$ | 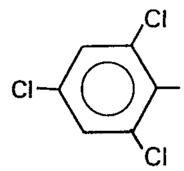 | 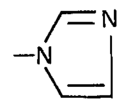 | Fp. 156-160°C |
| 13 | H | H | $Cl\text{-}CH_2\text{-}\overset{\overset{O}{\|}}{C}\text{-}$ | 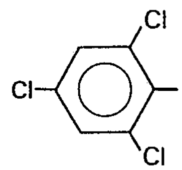 | 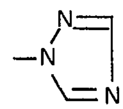 | Fp. 178°C |
| 14 | H | H | $CH_3\text{-}\overset{\overset{O}{\|}}{C}\text{-}$ | 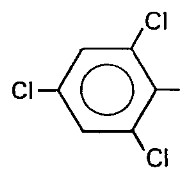 | 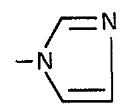 | Fp. 208-210°C |
| 15 | H | H | $(CH_3)_2\text{-}CH_2\overset{\overset{O}{\|}}{C}\text{-}$ | 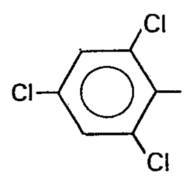 | 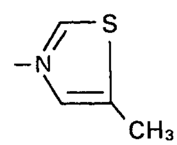 | Fp. 183-185°C |
| 16 | H | H | H | 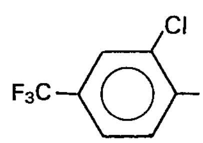 | 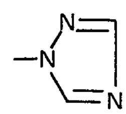 | Fp. 156-159°C |
| 17 | H | H | H | 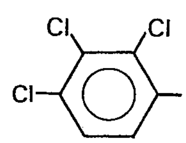 | 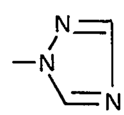 | Fp. 165°C |
| 18 | H | H | $CH_3\text{-}NH\text{-}\overset{\overset{O}{\|}}{C}\text{-}$ | 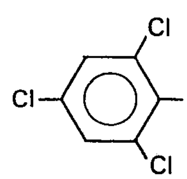 | 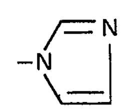 | Fp. 209-219°C |
| 19 | H | H | $Cl\text{-}CH_2\text{-}\overset{\overset{O}{\|}}{C}\text{-}$ | 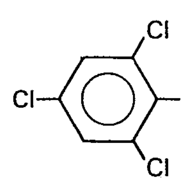 | 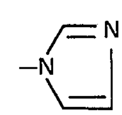 | Fp. 155-157°C |

Tabelle 2 (Fortsetzung)

| Bei-spiel Nr. | $R^1$ | $R^2$ | $R^3$ | 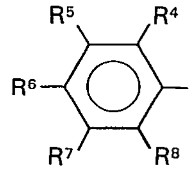 | Het | physikalische Eigenschaften |
|---|---|---|---|---|---|---|
| 20 | H | H | $CH_3O\text{-}CH_2\text{-}\overset{O}{\overset{\|}{C}}\text{-}$ | 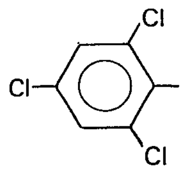 | 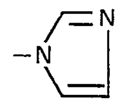 | Fp. 126-128°C |
| 21 | H | $CH_3NH\overset{O}{\overset{\|}{C}}\text{-}$ | $CH_3\text{-}NH\text{-}\overset{O}{\overset{\|}{C}}\text{-}$ | 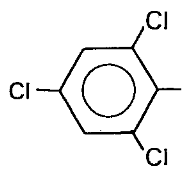 | 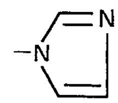 | Fp. 145-147°C |
| 22 | H | H | $Cl\text{-}CH_2\text{-}\overset{O}{\overset{\|}{C}}\text{-}$ | 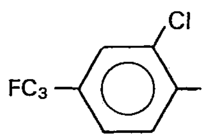 | 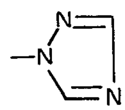 | Fp. 155-158°C |
| 23 | H | H | $(CH_3)_2\text{-}CH\text{-}\overset{O}{\overset{\|}{C}}\text{-}$ | 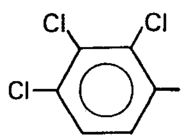 | 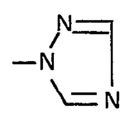 | Fp. >230°C |
| 24 | H | H | $C_2H_5\overset{O}{\overset{\|}{C}}\text{-}$ | 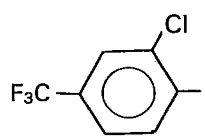 | 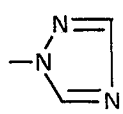 | Fp. 155-159°C |
| 25 | H | H | $C_2H_5\text{-}\overset{O}{\overset{\|}{C}}\text{-}$ | 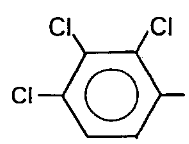 | 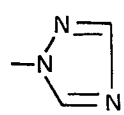 | Fp. 242-244°C |
| 26 | H | H | H | 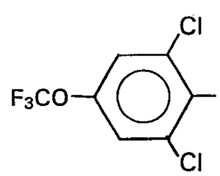 | 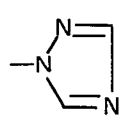 | Fp. 112-115°C |
| 27 | H | H | H | 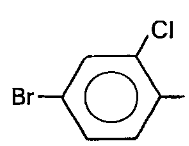 | 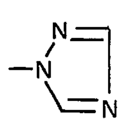 | Fp. 88-93°C |

Tabelle 2 (Fortsetzung)

| Bei-spiel Nr. | $R^1$ | $R^2$ | $R^3$ | 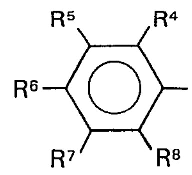 | Het | physikalische Eigenschaften |
|---|---|---|---|---|---|---|
| 28 | H | O<br>‖<br>$CH_3NH-C-$ | O<br>‖<br>$CH_3NH-C-$ | 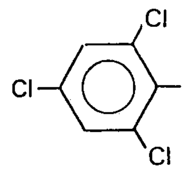 | 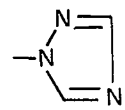 | Fp. 152-159°C |
| 29 | H | H | O<br>‖<br>$CH_3-C-$ | 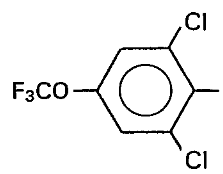 | 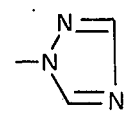 | Fp. 188-192°C |
| 30 | H | H | O<br>‖<br>$CH_3NH-C-$ | 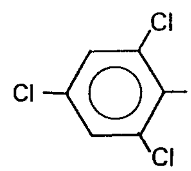 | 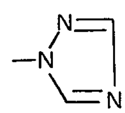 | Fp. 210-212°C |
| 31 | H | H | O<br>‖<br>$C_2H_5-C-$ | 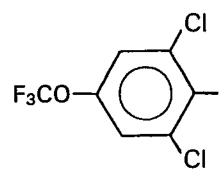 | 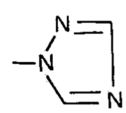 | Fp. 138-140°C |
| 32 | H | H | O<br>‖<br>$(CH_3)_2CH-C-$ | 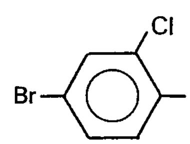 | 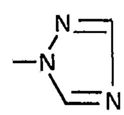 | Fp. 157-159°C |
| 33 | H | H | O<br>‖<br>$C_2H_5-C-$ | 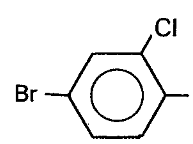 | 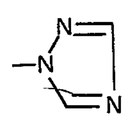 | Fp. 150-153°C |
| 34 | H | H | O<br>‖<br>$ClCH_2-C-$ | 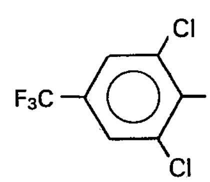 | 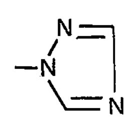 | Fp. 156-158°C |
| 35 | H | H | H | 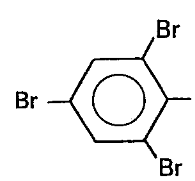 | 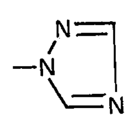 | Fp. 179-181°C |

Tabelle 2 (Fortsetzung)

| Bei-spiel Nr. | $R^1$ | $R^2$ | $R^3$ | 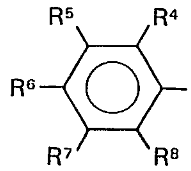 | Het | physikalische Eigenschaften |
|---|---|---|---|---|---|---|
| 36 | H | H | H | 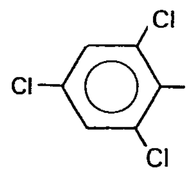 | 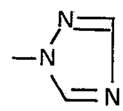 | Fp. 146-148°C |
| 37 | H | H | $(CH_3)_2CH-\overset{O}{\overset{\|}{C}}-$ | 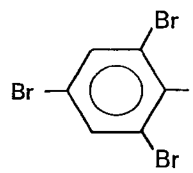 | 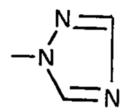 | Fp. 92-98°C |
| 38 | H | H | $C_2H_5-\overset{O}{\overset{\|}{C}}-$ | 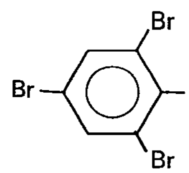 | 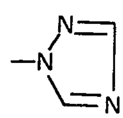 | Fp. 118-120°C |
| 39 | H | H | $C_2H_5-\overset{O}{\overset{\|}{C}}-$ | 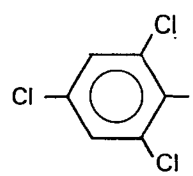 | 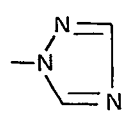 | Fp. 178-182°C |
| 40 | H | H | $Cl(CH_2)_3-\overset{O}{\overset{\|}{C}}-$ | 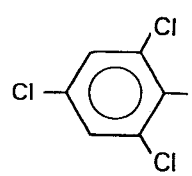 | 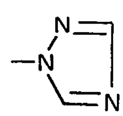 | Fp. 195-200°C |
| 41 | H | $C_2H_5-\overset{O}{\overset{\|}{C}}-$ | $CH_3-$ | 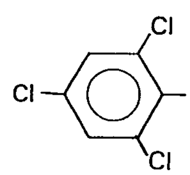 | 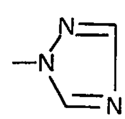 | Fp. 163°C |
| 42 | H | H | $CH_3-$ | 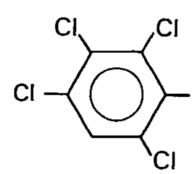 | 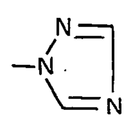 | Fp. 150-155°C |
| 43 | H | $C_2H_5-\overset{O}{\overset{\|}{C}}-$ | $CG_2=CH-CH_2-$ | 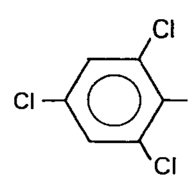 | 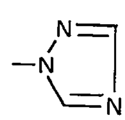 | Fp. 112°C |

Tabelle 2 (Fortsetzung)

| Bei-spiel Nr. | $R^1$ | $R^2$ | $R^3$ | 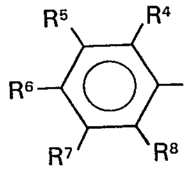 | Het | physikalische Eigenschaften |
|---|---|---|---|---|---|---|
| 44 | H | H | H | 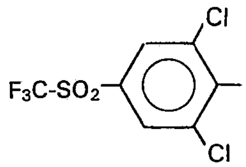 | 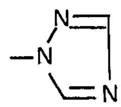 | Fp. 126-131°C |
| 45 | H | H | 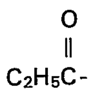 | 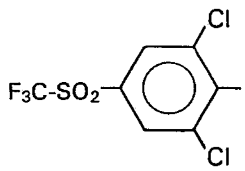 | 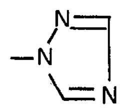 | Fp. 238°C |
| 46 | H | H | 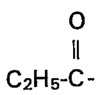 | 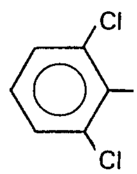 | 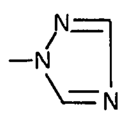 | Fp. 202-204°C |
| 47 | H | H | 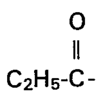 | 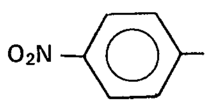 | 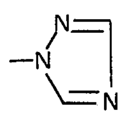 | Fp. 198-100°C |
| 48 | H | H | H | 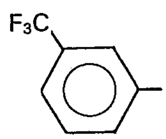 | 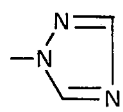 | Fp. 104°C |
| 49 | H | H | H | 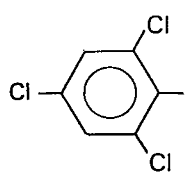 | 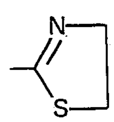 | Fp. 178-180°C |
| 50 | H | H | 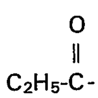 | 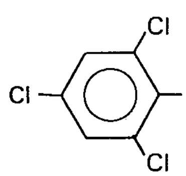 | 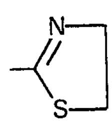 | Fp. 124°C |
| 51 | H | H | H | 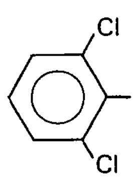 | 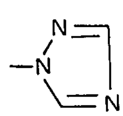 | Fp. 156-170°C |

Tabelle 2 (Fortsetzung)

| Bei-spiel Nr. | $R^1$ | $R^2$ | $R^3$ | 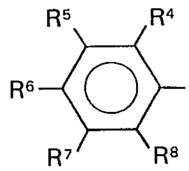 | Het | physikalische Eigenschaften |
|---|---|---|---|---|---|---|
| 52 | H | H | H | 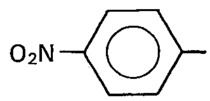 | 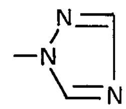 | Fp. 218-220°C |
| 53 | H | H | H | 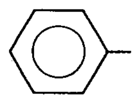 | 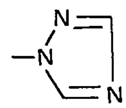 | Fp. 139°C |
| 54 | H | H | $C_2H_5-C(=O)-$ | 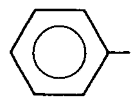 | 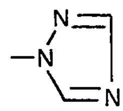 | Fp. 159-161°C |
| 55 | H | H | $C_2H_5-C(=O)-$ | 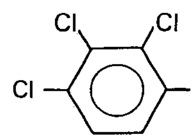 | 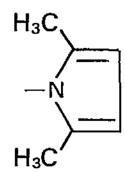 | Fp. 195°C |
| 56 | H | H | $C_2H_5-C(=O)-$ | 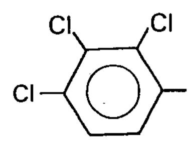 | 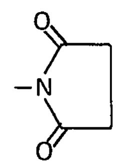 | Fp. 89-90°C |
| 57 | H | H | $CH_2=CH-CH_2-$ | 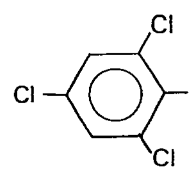 | 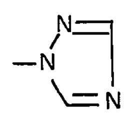 | |
| 58 | H | H | H | 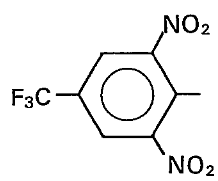 | 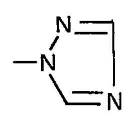 | Fp. 178-182°C |
| 59 | H | H | $C_2H_5-C(=O)-$ | 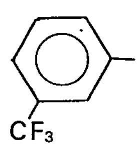 | 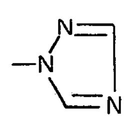 | Fp. 123-124°C |
| 60 | H | H | $Cl_2CH-C(=O)-$ | 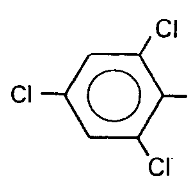 | 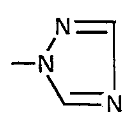 | |

Tabelle 2 (Fortsetzung)

| Bei-spiel Nr. | $R^1$ | $R^2$ | $R^3$ | 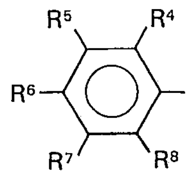 | Het | physikalische Eigenschaften |
|---|---|---|---|---|---|---|
| 61 | H | H | $C_2H_5$-CO- | 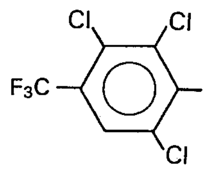 | 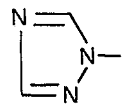 | Fp. 188-190°C |
| 62 | H | H | H | 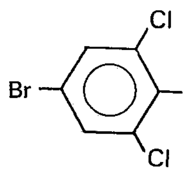 | 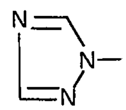 | Fp. 170°C |
| 63 | H | $C_2H_5$-CO | H | 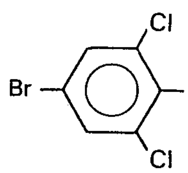 | 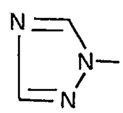 | Fp. 193°C |
| 64 | H | H | $CH_2=CH-CH_2$- | 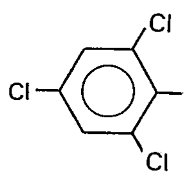 | 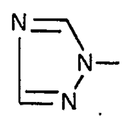 | Fp. 100-105°C |
| 65 | H | H | H | 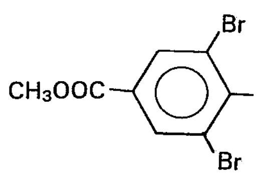 | 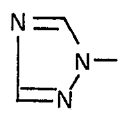 | Öl |
| 66 | H | H | $(CH_3)_2C=CH$-CO- | 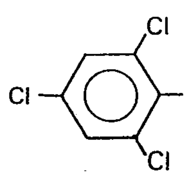 | 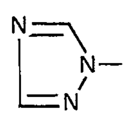 | Fp. 196-200°C |
| 67 | H | H | H | 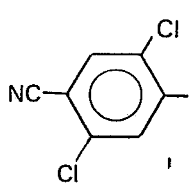 | 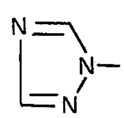 | Fp. 235°C |
| 68 | H | $CH_3-CH=CH$-CO- | $CH_3-CH=CH$-CO- | 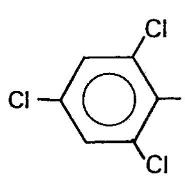 | 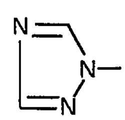 | Öl |

Tabelle 2 (Fortsetzung)

| Bei-spiel Nr. | $R^1$ | $R^2$ | $R^3$ | 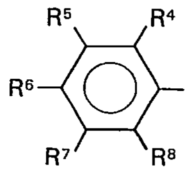 | Het | physikalische Eigenschaften |
|---|---|---|---|---|---|---|
| 69 | H | H | H | 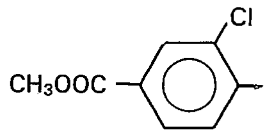 | 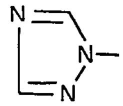 | Fp. 165-170°C |
| 70 | H | H | $C_2H_5CO$- | 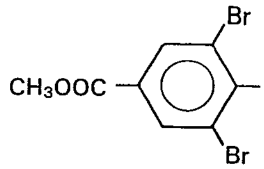 | 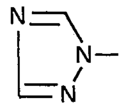 | Fp. 95-98°C |
| 71 | H | H | H | 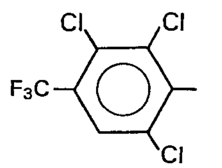 | 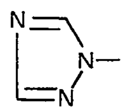 | Fp. 60-65°C |
| 72 | H | H | $ClCH_2$-CO | 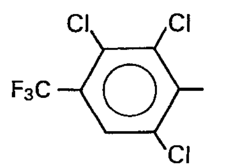 | 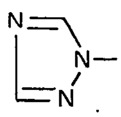 | Fp. 116-118°C |
| 73 | H | H | $C_2H_5CO$- | 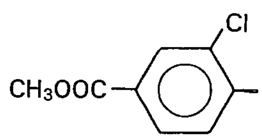 | 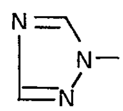 | Fp. 135-138°C |
| 74 | H | H | $C_2H_5CO$- | 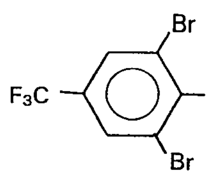 | 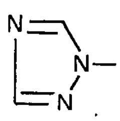 | Fp. 174°C |
| 75 | H | H | H | 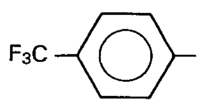 | 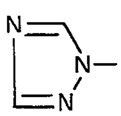 | Fp. 175-177°C |
| 76 | H | H | H | 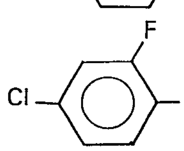 | 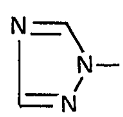 | Fp. 140-142°C |
| 77 | H | H | H | 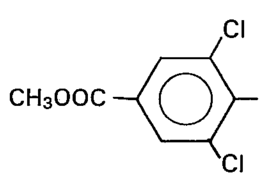 | 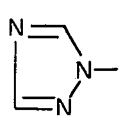 | Fp. 148°C |

Tabelle 2 (Fortsetzung)

| Beispiel Nr. | $R^1$ | $R^2$ | $R^3$ | 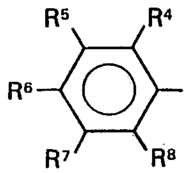 | Het | physikalische Eigenschaften |
|---|---|---|---|---|---|---|
| 78 | H | H | H | 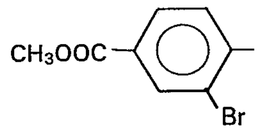 | 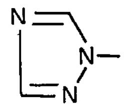 | Fp. 140-142°C |
| 79 | H | H | $C_2H_5CO$- | 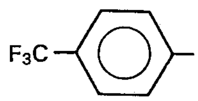 | 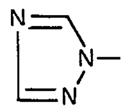 | Fp. 155-158°C |
| 80 | H | H | $ClCH_2$-CO- | 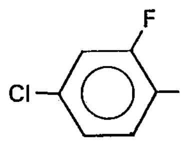 | 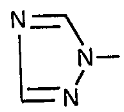 | Fp. 145°C |
| 81 | H | H | $C_2H_5CO$- | 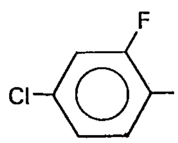 | 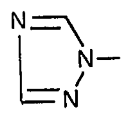 | Fp. 175°C |
| 82 | H | H | $C_2H_5CO$- | 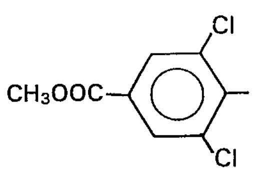 | 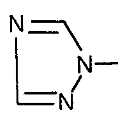 | Fp. 188°C |
| 83 | H | H | $F_3C$-CO- | 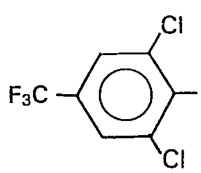 | 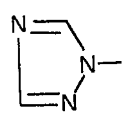 | Fp. 215-218°C |
| 84 | H | H | H | 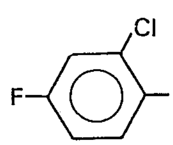 | 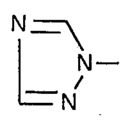 | Fp. 130-134°C |
| 85 | H | H | $C_2H_5$-CO- | 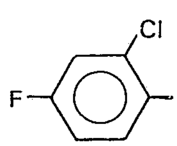 | 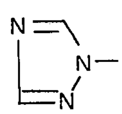 | Fp. 140-142°C |
| 86 | H | H | H | 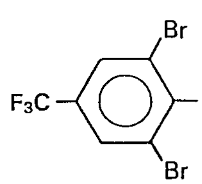 | 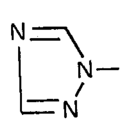 | Fp. 166°C |

Tabelle 2 (Fortsetzung)

| Bei-spiel Nr. | $R^1$ | $R^2$ | $R^3$ | 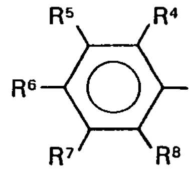 | Het | physikalische Eigenschaften |
|---|---|---|---|---|---|---|
| 87 | H | H | $C_2H_5$-CO- | 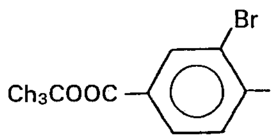 | 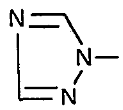 | Fp. 110-112°C |

*Anwendungsbeispiele:*

In den folgenden Anwendungsbeispielen wurde die nachstehend aufgeführte Verbindung als Vergleichssubstanz herangezogen:

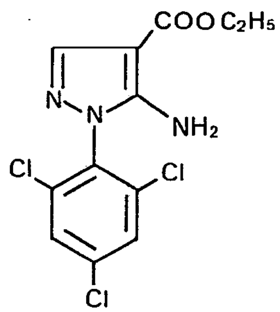

(A)

5-Amino-4-ethoxycarbonyl-1-(2,4,6-trichlorphenyl)-pyrazol

(Bekannt aus DE-OS 3 129 429)

*Beispiel A*

*Pre-emergence-Test*

Lösungsmittel: 5 Gewichtsteile Aceton
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

Eine deutliche Überlegenheit in der Wirksamkeit bei vergleichbarer Kulturpflanzenselektivität gegenüber dem Stand der Technik zeigen in diesem Test z.B. die Verbindung gemäss folgender Herstellungsbeispiele: 1 und 2.

*Beispiel B*

*Pre-emergence-Test*

Lösungsmittel: 5 Gewichtsteile Aceton
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, dass die jeweils gewünschten Wirkstoffmengen pro Flächeneinheit ausgebracht werden. Die Konzentration der Spritzbrühe wird so gewählt, dass in 200 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

Eine deutliche Überlegenheit in der Wirksamkeit bei vergleichbarer Kulturpflanzenselektivität zeigen in diesem Test z.B. die Verbindungen gemäss folgender Herstellungsbeispiele: 1 und 2.

*Beispiel C*

*Wuchshemmung bei Gerste*

Lösungsmittel: 30 Gewichtsteile Dimethylformamid
Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und

Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnass mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Eine deutliche Wirksamkeit in diesem Test gegenüber der unbehandelten Kontrolle zeigt beispielsweise die Verbindung gemäss Herstellungsbeispiel 5.

*Beispiel D*

*Entlaubung und Austrocknung der Blätter bei Baumwolle*

Lösungsmittel: 30 Gewichtsteile Dimethylformamid
Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnass mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
* leichtes Austrocknen der Blätter, geringer Blattfall
** starkes Austrocknen der Blätter, starker Blattfall
*** sehr starkes Austrocknen der Blätter. sehr starker Blattfall

Eine deutliche Wirksamkeit in diesem Test gegenüber der unbehandelten Kontrolle zeigt beispielsweise die Verbindung gemäss Herstellungsbeispiel 6.

**Patentansprüche**

1. 5-Amino-4-heterocyclyl-1-phenylpyrazole der allgemeinen Formel (I)

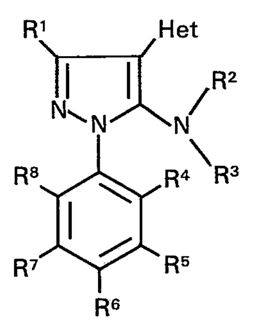

(I)

in welcher

$R^1$ für Wasserstoff oder für geradkettiges oder verzweigtes Alkyl mit 1 bis 8 Kohlenstoffatomen steht,

$R^2$ für Wasserstoff oder einen Rest $-\underset{\underset{X}{\|}}{C}-R^9$ steht,

$R^3$ unabhängig von $R^2$ für die gleichen Reste wie $R^2$ steht und zusätzlich für jeweils geradkettiges oder verzweigtes Alkyl, Alkenyl oder Alkinyl mit bis zu 4 Kohlenstoffatomen steht,

$R^4$, $R^5$, $R^6$, $R^7$ und $R^8$ unabhängig voneinander für Wasserstoff, Cyano, Nitro, Halogen oder für jeweils geradkettiges oder verzweigtes Alkyl, Alkoxy oder Alkoxycarbonyl mit jeweils bis zu 4 Kohlenstoffatomen, ausserdem für jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder für einen Rest $-S(O)_n-R^{10}$ stehen, wobei

$R^9$ für Wasserstoff, für jeweils geradkettiges oder verzweigtes Alkyl, Alkenyl, Alkinyl, Alkoxyalkyl, Alkylthioalkyl, Alkoxy, Alkylthio, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen, ausserdem für gegebenenfalls einfach oder mehrfach, gleich oder verschieden durch Halogen, niederes Alkyl oder niederes Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenoxyalkyl, Phenylthio oder Phenylamino steht, wobei als Phenylsubstituenten die bei $R^9$ genannten infrage kommen;

$R^{10}$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,

X für Sauerstoff oder Schwefel steht.

n für eine Zahl 0, 1 oder 2 steht und

Het für einen gegebenenfalls einfach oder mehrfach, gleich oder verschieden substituierten, gesättigten oder ungesättigten, fünf- oder sechsgliedrigen Heterocyclus steht, der ein bis drei gleiche oder verschiedene Heteroatome aus der Gruppe Stickstoff, Sauerstoff und Schwefel enthält und der über ein Kohlenstoff- oder ein Stickstoffatom verknüpft sein kann, wobei als Substituenten infrage kommen: Halogen, Nitro, sowie jeweils geradkettiges oder verzweigtes Alkyl, Alkoxy, Alkylthio oder Halogenalkyl mit jeweils bis zu 4 Kohlenstoffatomen und gegebenenfalls bis zu 9 gleichen oder verschiedenen Halogenatomen,

2. Verfahren zur Herstellung von 5-Amino-4-heterocyclyl-1-phenylpyrazolen der allgemeinen Formel (1)

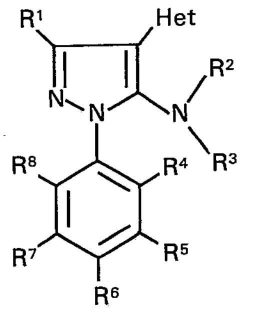

(I)

in welcher

$R^1$ für Wasserstoff oder für geradkettiges oder verzweigtes Alkyl mit 1 bis 8 Kohlenstoffatomen steht,

$R^2$ für Wasserstoff oder einen Rest $-\underset{\underset{X}{\|}}{C}-R^9$ steht,

$R^3$ unabhängig von $R^2$ für die gleichen Reste wie $R^2$ steht und zusätzlich für jeweils geradkettiges oder verzweigtes Alkyl, Alkenyl oder Alkinyl mit bis zu 4 Kohlenstoffatomen steht,

$R^4$, $R^5$, $R^6$, $R^7$ und $R^8$ unabhängig voneinander für Wasserstoff, Cyano, Nitro, Halogen oder für jeweils geradkettiges oder verzweigtes Alkyl, Alkoxy oder Alkoxycarbonyl mit jeweils bis zu 4 Kohlenstoffatomen, ausserdem für jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder für einen Rest $-S(O)_n-R^{10}$ stehen, wobei

$R^9$ für Wasserstoff, für jeweils geradkettiges oder verzweigtes Alkyl, Alkenyl, Alkinyl, Alkoxyalkyl, Alkylthioalkyl, Alkoxy, Alkylthio, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen, ausserdem für gegebenenfalls einfach oder mehrfach, gleich oder verschieden durch Halogen, niederes Alkyl oder niederes Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenoxyalkyl, Phenylthio oder Phenylamino steht, wobei als Phenylsubstituenten die bei $R^9$ genannten infrage kommen;

$R^{10}$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,

X für Sauerstoff oder Schwefel steht.

n für eine Zahl 0, 1 oder 2 steht und

Het für einen gegebenenfalls einfach oder mehrfach, gleich oder verschieden substituierten, gesättigten oder ungesättigten, fünf- oder sechsgliedrigen Heterocyclus steht, der ein bis drei gleiche oder verschiedene Heteroatome aus der Gruppe Stickstoff, Sauerstoff und Schwefel enthält und der über ein Kohlenstoff- oder ein Stickstoffatom verknüpft sein kann, wobei als Substituenten infrage kommen: Halogen, Nitro, sowie jeweils geradkettiges oder verzweigtes Alkyl, Alkoxy, Alkylthio oder Halogenalkyl mit jeweils bis zu 4 Kohlenstoffatomen und gegebenenfalls bis zu 9 gleichen oder verschiedenen Halogenatomen,

dadurch gekennzeichnet, dass man

(a) Phenylhydrazine der Formel (II),

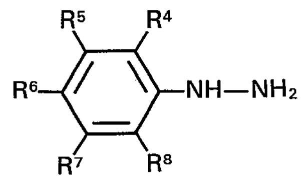

(II)

in welcher

$R^4$, $R^5$, $R^6$, $R^7$ und $R^8$ die oben angegebene Bedeutung haben,

mit Acrylnitril-Derivaten der Formel (III),

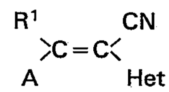

(III)

in welcher

$R^1$ und Het die oben angegebene Bedeutung haben und

A für Chlor, Brom, Methoxy, Ethoxy oder Dimethylamino steht,

entweder zunächst in einer 1. Stufe, gegebenenfalls in Gegenwart eines Verdünnungsmittels sowie gegebenenfalls in Gegenwart eines Reaktionshilfsmittels, umsetzt zu den Phenylhydrazin-Derivaten der Formel (IV),

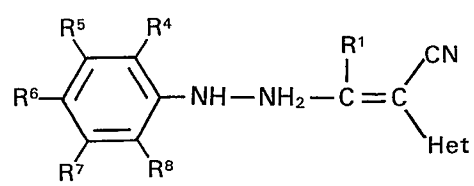

(IV)

in welcher

$R^1$, $R^4$, $R^5$, $R^6$, $R^7$, $R^8$ und Het die oben angegebene Bedeutung haben,

und diese in einer 2. Stufe, gegebenenfalls in Gegenwart eines Verdünnungsmittels und gegebenenfalls in Gegenwart eines sauren Katalysators, cyclisiert oder direkt in einem Reaktionsschritt ohne Isolierung der Zwischenstufe der Formel (IV), gegebenenfalls in Gegenwart eines Verdünnungsmittels, cyclisiert zu den 5-Amino-pyrazolon der Formel (Ia),

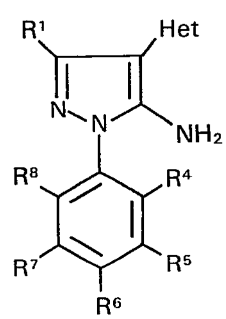

(Ia)

in welcher
$R^1$, $R^4$, $R^5$, $R^6$, $R^7$, $R^8$ und Het die oben angegebene Bedeutung haben,
oder dass man
(b) die nach Verfahren (a) erhältlichen 5-Amino-pyrazole der Formel (Ia)

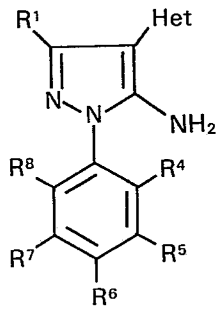

(Ia)

in welcher
$R^1$, $R^4$, $R^5$, $R^6$, $R^7$, $R^8$ und Het die oben angegebene Bedeutung haben,
in allgemein üblicher Weise mit Acylierungsmitteln oder Alkylierungsmitteln der Formel (V)

$$R^{11} - A' \qquad (V)$$

in welcher
$R^{11}$ für jeweils geradkettiges oder verzweigtes Alkyl, Alkenyl oder Alkinyl mit bis zu 4 Kohlenstoffatomen oder einen Rest
X
‖
$-C-R^9$ steht, wobei X und $R^9$ die oben angegebene Bedeutung haben und
A′ für Chlor, Brom, Jod, p-Toluolsulfonyloxy, Alkoxysulfonyloxy oder Acyloxy steht,
oder mit Iso(thio)cyanaten der Formel (VI),

$$R^{12} - N = C = X \qquad (VI)$$

in welcher
$R^{12}$ für geradkettiges oder verzweigtes Alkyl mit bis zu 4 Kohlenstoffatomen, oder für gegebenenfalls ein- bis dreifach, gleich oder verschieden substituiertes Phenyl steht, wobei als Substituenten infrage kommen: Halogen, jeweils geradkettiges oder verzweigtes Alkyl, Alkoxy oder Halogenalkyl mit jeweils bis zu 4 Kohlenstoffatomen und im Fall des Halogenalkyl mit 1 bis zu 9 gleichen oder verschiedenen Halogenatomen, und
X die oben angegebene Bedeutung hat,
gegebenenfalls in Gegenwart eines Verdünnungsmittels und gegebenenfalls in Gegenwart eines Säurebindemittels, umsetzt zu den am Stickstoff alkylierten bzw. acylierten 5-Amino-pyrazolen der Formel (Ib),

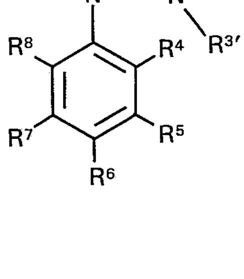

(Ib)

in welcher
$R^1$, $R^2$, $R^4$, $R^5$, $R^6$, $R^7$, $R^8$ und Het die oben angegebene Bedeutung haben und
$R^{3'}$ für die gleichen Reste, wie das oben angegebene $R^3$ mit Ausnahme des Wasserstoffrestes steht.

3. Herbizide und pflanzenwuchsregulierende Mittel, gekennzeichnet durch einen Gehalt an mindestens einem 5-Amino-4-heterocyclyl-1-phenyl-pyrazol der Formel (I) gemäss Anspruch 1 und 2.

4. Verwendung von 5-Amino-4-heterocyclyl-1--phenylpyrazolen der Formel (I) gemäss Anspruch 1 und 2 zur Bekämpfung von unerwünschtem Pflanzenwachstum und/oder als Pflanzenwachstumsregulatoren.

5. Verfahren zur Herstellung von herbiziden und/oder pflanzenwuchsregulierenden Mitteln, dadurch gekennzeichnet, dass man 5-Amino-4-heterocyclyl-1-phenylpyrazole der allgemeinen Formel (I) gemäss Anspruch 1 und 2 mit Streckmitteln und/oder oberflächenaktiven Mitteln vermischt.

6. Acrylnitril-Derivate der Formel (III),

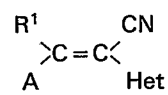

(III)

in welcher
$R^1$ für Wasserstoff oder für geradkettiges oder verzweigtes Alkyl mit 1 bis 8 Kohlenstoffatomen steht,
Het für einen gegebenenfalls einfach oder mehrfach, gleich oder verschieden unsubstituierten, gesättigten oder ungesättigten, fünf- oder sechsgliedrigen Heterocyclus steht, der ein bis drei gleiche oder verschiedene Heteroatome aus der Gruppe Stickstoff, Sauerstoff oder Schwefel enthält und der über ein Kohlenstoff- oder ein Stickstoffatom verknüpft sein kann, wobei als Subtituenten infrage kommen: Halogen, Nitro, sowie jeweils geradkettiges oder verzweigtes Alkyl, Alkoxy, Alkylthio oder Halogenalkyl mit jeweils bis zu 4 Kohlenstoffatomen und gegebenenfalls bis zu 9 gleichen oder verschiedenen Halogenatomen und
A für Chlor, Brom, Methoxy, Ethoxy oder Dimethylamino steht.

7. Verfahren zur Herstellung von Acrylnitril-Derivaten der Formel (III),

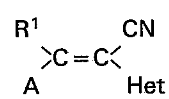

(III)

in welcher
$R^1$ für Wasserstoff oder für geradkettiges oder verzweigtes Alkyl mit 1 bis 8 Kohlenstoffatomen steht,
Het für einen gegebenenfalls einfach oder mehrfach, gleich oder verschieden unsubstituierten, gesättigten oder ungesättigten, fünf- oder sechsgliedrigen Heterocyclus steht, der ein bis drei gleiche oder verschiedene Heteroatome aus der Gruppe Stickstoff, Sauerstoff und Schwefel enthält und der über ein Kohlenstoff- oder ein Stickstoffatom verknüpft sein kann, wobei als Subtituenten infrage kommen: Halogen, Nitro, sowie jeweils geradkettiges oder verzweigtes Alkyl, Alkoxy, Alkylthio oder Halogenalkyl mit jeweils bis zu 4 Kohlenstoffatomen und gegebenenfalls bis zu 9 gleichen oder verschiedenen Halogenatomen und
A für Chlor, Brom, Methoxy, Ethoxy oder Dimethylamino steht,
dadurch gekennzeichnet, dass man
(1) für den Fall, dass A für Methoxy oder Ethoxy steht,
Acetonitril-Derivate der Formel (VIII)

$$Het\text{-}CH_2\text{-}CN \qquad (VIII)$$

in welcher
Het die oben angegebene Bedeutung hat,
mit Orthoestern der Formel (IX),

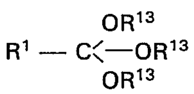
(IX)

in welcher
$R^1$ die oben angegebene Bedeutung hat und
$R^{13}$ für Methyl oder Ethyl steht,
gegebenenfalls in Gegenwart eines Verdünnungsmittels bei Temperaturen zwischen +80°C und +200°C umsetzt,
oder dass man
(2) für den Fall, dass A für Dimethylamino steht, Acetonitril-Derivate der Formel (VIII)

$$Het\text{-}CH_2\text{-}CN$$

in welcher
Het die oben angegebene Bedeutung hat,
mit Amidacetalen der Formel (X)

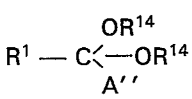
(X)

in welcher
$R^1$ die oben angegebene Bedeutung hat,
$R^{14}$ für Methyl oder Ethyl steht und
A'' für Dimethylamino steht,
gegebenenfalls in Gegenwart eines Verdünnungsmittels bei Temperaturen zwischen +80°C und +200°C umsetzt, oder dass man
(3) für den Fall, dass A für Hydroxy steht, Acetonitril-Derivate der Formel (VIII),

$$Het\text{-}CH_2\text{-}CN \qquad (VIII)$$

in welcher
Het die oben angegebene Bedeutung hat,
mit Estern der Formel (XI),

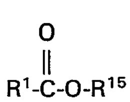
(XI)

in welcher
$R^1$ die oben angegebene Bedeutung hat und
$R^{15}$ für Methyl oder Ethyl steht,
gegebenenfalls in Gegenwart eines Verdünnungsmittels und gegebenenfalls in Gegenwart eines basischen Katalysators bei Temperaturen zwischen 0°C und 100°C umsetzt,
oder dass man
(4) für den Fall, dass A für Chlor oder Brom steht, die nach Verfahren (3) erhältlichen Hydroxyverbindungen der Formel (IIIa),

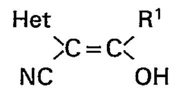
(IIIa)

in welcher
$R^1$ und Het die oben angegebene Bedeutung haben,
mit Halogenierungsmitteln bei Temperaturen zwischen 0°C und 100°C umsetzt.

## Claims

1. 5-Amino-4-heterocyclyl-1-phenylpyrazoles of the general formula (I)

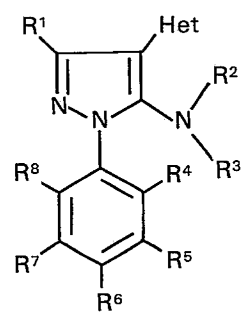
(I)

in which
$R^1$ represents hydrogen or straight-chain or branched alkyl having 1 to 8 carbon atoms,
$R^2$ represents hydrogen or a radical 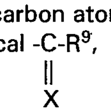
$R^3$ independently of $R^2$ represents the same radicals as $R^2$, and additionally represents, in each case straight-chain or branched, alkyl, alkenyl or alkynyl having up to 4 carbon atoms, $R^4$, $R^5$, $R^6$, $R^7$ and $R^8$ independently of one another represent hydrogen, cyano, nitro or halogen, or represent, in each case straight-chain or branched, alkyl, alkoxy or alkoxycarbonyl having in each case up to 4 carbon atoms, or moreover represent, in each case straight-chain or branched, halogenoalkyl or halogenoalkoxy having in each case up to 4 carbon atoms and up to 9 identical or different halogen atoms, or represent a radical $-S(O)_n-R^{10}$,
wherein
$R^9$ represents hydrogen, or represents, in each case straight-chain or branched, alkyl, alkenyl, alkynyl, alkoxyalkyl, alkylthioalkyl, alkoxy, alkylthio, alkylamino, dialkylamino or halogenoalkyl having in each case up to 4 carbon atoms in the individual alkyl parts, and in the case of

halogenoalkyl having up to 9 identical or different halogen atoms, or moreover represents cycloalkyl which has 3 to 7 carbon atoms and is optionally mono- or polysubstituted, identically or differently, by halogen, lower alkyl or lower halogenoalkyl, or represents phenyl, phenoxy, phenoxyalkyl, phenylthio or phenylamino which is in each case optionally mono- or polysubstituted, identically or differently, by vatious substituents, possible phenyl substituents being those mentioned for $R^9$;

$R^{10}$ represents amino, or represents, in each case straight-chain or branched, alkyl, alkylamino, dialkylamino or halogenoalkyl having in each case up to 4 carbon atoms in the individual alkyl parts, and in the case of halogenoalkyl having up to 9 identical or different halogen atoms,

X represents oxygen or sulphur,

n represents the number 0, 1 or 2 and

Het represents a saturated or unsaturated five-membered or six-membered heterocyclic radical which is optionally mono- or polysubstituted, identically or differently, and which contains up to three identical or different hetero atoms from the group nitrogen, oxygen and sulphur and which can be linked via a carbon atom or a nitrogen atom, possible substituents being halogen, nitro and in each case straight-chain or branched alkyl, alkoxy, alkylthio or halogenoalkyl having in each case up to 4 carbon atoms and optionally up to 9 identical or different halogen atoms.

2. Process for the preparation of 5-amino-4-heterocyclyl-1-phenylpyrazoles of the general formula (I)

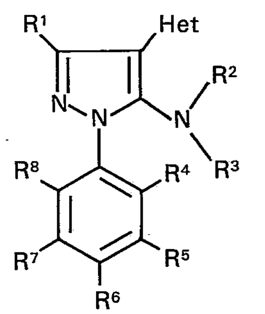

(I)

in which

$R^1$ represents hydrogen or straight-chain or branched alkyl having 1 to 8 carbon atoms,

$R^2$ represents hydrogen or a radical $-\underset{\underset{X}{\|}}{C}-R^9$,

$R^3$ independently of $R^2$ represents the same radicals as $R^2$, and additionally represents, in each case straight-chain or branched, alkyl, alkenyl or alkynyl having up to 4 carbon atoms,

$R^4$, $R^5$, $R^6$, $R^7$ and $R^8$ independently of one another represent hydrogen, cyano, nitro or halogen, or represent, in each case straight-chain or branched, alkyl, alkoxy or alkoxycarbonyl having in each case up to 4 carbon atoms, or moreover represent, in each case straight-chain or branched, halogenoalkyl or halogenoalkoxy having in each case up to 4 carbon atoms and up to 9 identical or different halogen atoms, or represent a radical $-S(O)_n-R^{10}$,

wherein

$R^9$ represents hydrogen, or represents, in each case straight-chain or branched, alkyl, alkenyl, alkynyl, alkoxyalkyl, alkylthioalkyl, alkoxy, alkylthio, alkylamino, dialkylamino or halogenoalkyl having in each case up to 4 carbon atoms in the individual alkyl parts, and in the case of halogenoalkyl having up to 9 identical or different halogen atoms, or moreover represents cycloalkyl which has 3 to 7 carbon atoms and is optionally mono- or polysubstituted, identically or differently, by halogen, lower alkyl or lower halogenoalkyl, or represents phenyl, phenoxy, phenoxyalkyl, phenylthio or phenylamino which is in each case optionally mono- or polysubstituted, identically or differently, by various substituents, possible phenyl substituents being those mentioned for $R^9$;

$R^{10}$ represents amino, or represents, in each case straight-chain or branched, alkyl, alkylamino, dialkylamino or halogenoalkyl having in each case up to 4 carbon atoms in the individual alkyl parts, and in the case of halogenoalkyl having up to 9 identical or different halogen atoms,

X represents oxygen or sulphur,

n represents the number 0, 1 or 2 and

Het represents a saturated or unsaturated five-membered or six-membered heterocyclic radical which is optionally mono- or polysubstituted, identically or differently, and which contains up to three identical or different hetero atoms from the group nitrogen, oxygen and sulphur and which can be linked via a carbon atom or a nitrogen atom, possible substituents being halogen, nitro and in each case straight-chain or branched alkyl, alkoxy, alkylthio or halogenoalkyl having in each case up to 4 carbon atoms and optionally up to 9 identical or different halogen atoms,

characterized in that

(a) phenylhydrazines of the formula (II),

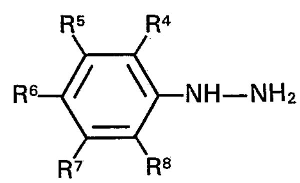

(II)

in which

$R^4$, $R^5$, $R^6$, $R^7$ and $R^8$ have the abovementioned meaning,

and acrylonitrile derivatives of the formula (III)

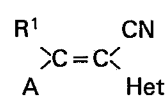

(III)

in which

$R^1$ and Het have the abovementioned meaning and

A represents chlorine, bromine, methoxy, ethoxy or dimethylamino,

either are reacted initially in a 1st stage, if appropri-

ate in the presence of a diluent and if appropriate in the presence of a reaction auxiliary, to give the phenylhydrazine derivatives of the formula (IV)

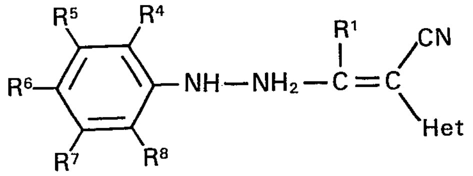
(IV)

in which
$R^1$, $R^4$, $R^5$, $R^6$, $R^7$, $R^8$ and Het have the abovementioned meaning,
and these are cyclized in a 2nd stage, if appropriate in the presence of a diluent and if appropriate in the presence of an acid catalyst, or are cyclized directly in one reaction step, without isolation of the intermediate of the formula (IV), if appropriate in the presence of a diluent, to give the 5-amino-pyrazoles of the formula (Ia)

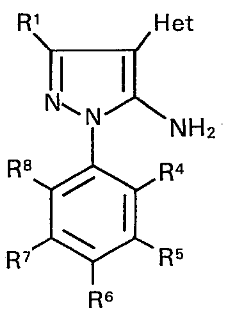
(Ia)

in which
$R^1$, $R^4$, $R^5$, $R^6$, $R^7$, $R^8$ and Het have the abovementioned meaning,
or in that
(b) the 5-amino-pyrazoles. obtainable according to process (a), of the formula (Ia)

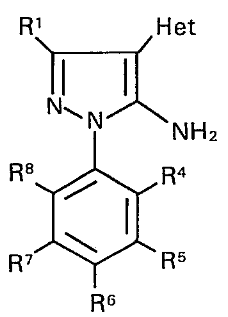
(Ia)

in which
$R^1$, $R^4$, $R^5$, $R^6$, $R^7$, $R^8$ and Het and the abovementioned meaning,
are reacted in the generally customary manner with acylating agents or alkylating agents of the formula (V)

$R^{11}$-A' (V)

in which
$R^{11}$ represents in each case straight-chain or branched alkyl, alkenyl or alkinyl with up to 4 carbon atoms or a

radical 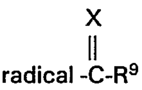

wherein
X and $R^9$ have the abovementioned meaning, and
A' represents chlorine, bromine, iodine, p-toluenesulphonyloxy, alkoxysulphonyloxy or acyloxy,
or with iso(thio)cyanates of the formula (VI)

$R^{12}$-N=C=X (VI)

in which
$R^{12}$ represents straight-chain or branched alkyl having up to 4 carbon atoms, or represents phenyl which is optionally mono-, di- or trisubstituted identically or differently, possible substituents being halogen, in each case straight-chain or branched alkyl, alkoxy or halogenoalkyl having in each case up to 4 carbon atoms, and in the case of halogenoalkyl having up to 9 identical or different halogen atoms and
X has the abovementioned meaning,
if appropriate in the presence of diluent and if appropriate in the presence of an acid-binding agent, to give the 5-amino-pyrazoles, alkylated or acylated on the nitrogen, of the formula (Ib)

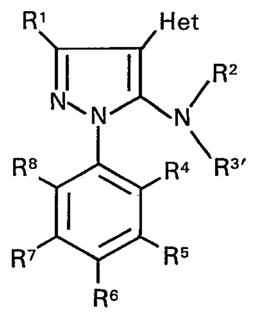
(Ib)

in which
$R^1$, $R^2$, $R^4$, $R^5$, $R^6$, $R^7$, $R^8$ and Het have the abovementioned meaning and
$R^{3'}$ represents the same radicals as the abovementioned $R^3$, with the exception of the hydrogen radical.

3. Herbicidal and plant growth-regulating agents, characterized in that they contain at least one 5-amino-4-heterocyclyl-1-phenylpyrazole of the formula (I) according to Claim 1 and 2.

4. Use of 5-amino-4-heterocyclyl-1-phenylpyrazoles of the formula (I) according to Claim 1 and 2 for combating undesirable plant growth and/or as plant growth regulators.

5. Process for the preparation of herbicidal and/or plant growth-regulating agents, characterized in that 5-amino-4-heterocyclyl-1-phenylpyrazoles of the general formula (I) according to Claim 1 and 2 are mixed with extenders and/or surface-active agents.

6. Acrylonitrile derivatives of the formula (III),

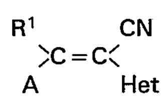
(III)

in which

$R^1$ represents hydrogen or straight-chain or branched alkyl having 1 to 8 carbon atoms,

Het represents a saturated or unsaturated five-membered or six-membered heterocyclic radical which is optionally mono- or polysubstituted, identically or differently, and which contains up to three identical or different hetero atoms from the group nitrogen, oxygen and sulphur and which can be linked via a carbon atom or a nitrogen atom, possible substituents being halogen, nitro and in each case straight-chain or branched alkyl, alkoxy, alkylthio or halogenoalkyl having in each case up to 4 carbon atoms and optionally up to 9 identical or different halogen atoms and

A represents chlorine, bromine, methoxy, ethoxy or dimethylamino.

7. Process for the preparation of acrylonitrile derivatives of the formula (III),

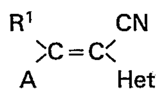 (III)

in which

$R^1$ represents hydrogen or straight-chain or branched alkyl having 1 to 8 carbon atoms,

Het represents a saturated or unsaturated five-membered or six-membered heterocyclic radical which is optionally mono- or polysubstituted, identically or differently, and which contains up to three identical or different hetero atoms from the group nitrogen, oxygen and sulphur and which can be linked via a carbon atom or a nitrogen atom, possible substituents being halogen, nitro and in each case straight-chain or branched alkyl, alkoxy, alkylthio or halogenoalkyl having in each case up to 4 carbon atoms and optionally up to 9 identical or different halogen atoms and

A represents chlorine, bromine, methoxy, ethoxy or dimethylamino,

characterized in that

(1) where A reresents methoxy or ethoxy, acetonitrile derivatives of the formula (VIII)

$$Het\text{-}CH_2\text{-}CN \qquad (VIII)$$

in which

Het has the abovementioned meaning

are reacted with orthoesters of the formula (IX),

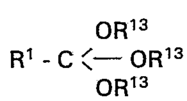 (IX)

in which

$R^1$ has the abovementioned meaning and

$R^{13}$ represents methyl or ethyl,

if appropriate in the presence of a diluent, at temperatures between +80°C and +200°C, or

(2) where A represents dimethylamino, acetonitrile derivatives of the formula (VIII)

$$Het\text{-}CH_2\text{-}CN$$

in which

Het has the abovementioned meaning,

are reacted with amidoacetals of the formula (X)

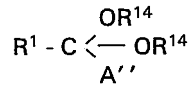 (X)

in which

$R^1$ has the abovementioned meaning,

$R^{14}$ represents methyl or ethyl and

A'' represents dimethylamino,

if appropriate in the presence of a diluent, at temperatures between +80°C and +200°C, or

(3) where A represents hydroxyl, acetonitrile derivatives of the formula (VIII),

$$Het\text{-}CH_2\text{-}CN \qquad (VIII)$$

in which

Het has the abovementioned meaning,

are reacted with esters of the formula (XI),

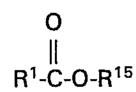 (XI)

in which

$R^1$ has the abovementioned meaning and

$R^{15}$ represents methyl or ethyl,

if appropriate in the presence of a diluent, and if appropriate in the presence of a basic catalyst, at temperatures between 0°C and 100°C, or

(4) where A represents chlorine or bromine, the hydroxy compounds, obtainable according to process (3), of the formula (IIIa),

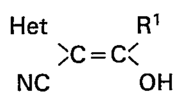 (IIIa)

in which

$R^1$ and Het have the abovementioned meaning,

are reacted with halogenating agents at temperatures between 0°C and 100°C.

## Revendications

1. 5-amino-4-hétérocyclyl-1-phénylpyrazole de formule générale (I)

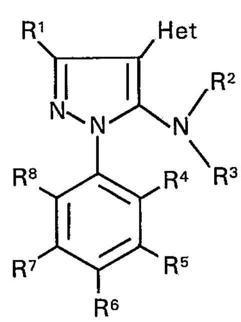 (I)

dans laquelle

$R^1$ représente l'hydrogène ou un groupe alkyle à chaîne droite ou ramifiée ayant 1 à 8 atomes de carbone,

$R^2$ représente l'hydrogène ou un reste $-\underset{\underset{X}{\|}}{C}-R^9$,

$R^3$ représente indépendamment de $R^2$ les mêmes restes que $R^2$ et représente en outre un groupe alkyle, alcényle ou alcynyle chacun à chaîne droite ou à chaîne ramifiée, avec jusqu'à 4 atomes de carbone,

$R^4$, $R^5$, $R^6$, $R^7$ et $R^8$ représentent indépendamment les uns des autres l'hydrogène, un groupe cyano, nitro, un halogène ou un groupe alkyle, alkoxy ou alkoxycarbonyle, chacun à chaîne droite ou à chaîne ramifiée, avec dans chaque cas jusqu'à 4 atomes de carbone, en outre un groupe halogénalkyle ou halogénalkoxy, chacun à chaîne droite ou ramifiée, avec dans chaque cas jusqu'à 4 atomes de carbone et jusqu'à 9 atomes d'halogènes identiques ou différents, ou un reste $(S(O)_n-R^{10}$,

$R^9$ représente l'hydrogène, un groupe alkyle, alcényle, alcynyle, alkoxyalkyle, alkylthioalkyle, alkoxy, alkylthio, alkylamino, dialkylamino ou halogénalkyle, chacun à chaîne droite ou à chaîne ramifiée, avec dans chaque cas jusqu'à 4 atomes de carbone dans les parties alkyle individuelles et dans le cas du groupe halogénalkyle, avec jusqu'à 9 atomes d'halogènes, identiques ou différents, en outre un groupe cycloalkyle de 3 à 7 atomes de carbone portant éventuellement un ou plusieurs substituants halogéno, alkyle inférieur ou halogénalkyle inférieur identiques ou différents, ainsi qu'un groupe phényle, phénoxy, phénoxyalkyle, phénylthio ou phénylamino, chacun portant le cas échéant un ou plusieurs substituants identiques ou différents, et on considère alors comme substituants du groupe phényle les substituants mentionnés dans le cas de $R^9$;

$R^{10}$ est un groupe amino, ainsi qu'un groupe alkyle, alkylamino, dialkylamino ou halogénalkyle, chacun à chaîne droite ou à chaîne ramifiée et avec dans chaque cas jusqu'à 4 atomes de carbone dans les parties individuelles, et dans le cas du groupe halogénalkyle, avec jusquà 9 atomes d'halogènes identiques ou différents,

X est l'oxygène ou le soufre,

n est le nombre 0, 1 ou 2 et

Het désigne un hétérocycle pentagonal ou hexagonal saturé ou non saturé, portant éventuellement un ou plusieurs substituants identiques ou différents, qui comprend un à trois hétéroatomes identiques ou différents du groupe formé par l'azote, l'oxygène et le soufre et qui peut être lié par l'intermédiaire d'un atome de carbone ou d'un atome d'azote, et on considère alors comme substituants: un halogène, un groupe nitro, ainsi qu'un groupe alkyle, alkoxy, alkylthio ou halogénalkyle chacun à chaîne droite ou à chaîne ramifiée avec, dans chaque cas, jusqu'à 4 atomes de carbone et, le cas échéant, jusqu'à 9 atomes d'halogènes identiques ou différents.

2. Procédé de préparation de 5-amino-4-hétérocyclyl-1-phénylpyrrazoles de formule générale (I)

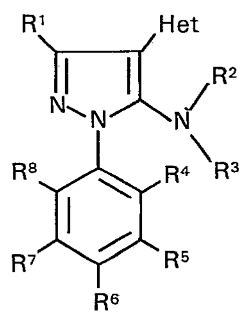

(I)

dans laquelle

$R^1$ représente l'hydrogène ou un groupe alkyle à chaîne droite ou ramifiée ayant 1 à 8 atomes de carbone,

$R^2$ représente l'hydrogène ou un reste $-\underset{\underset{X}{\|}}{C}-R^9$,

$R^3$ représente indépendamment de $R^2$ les mêmes restes que $R^2$ et représente en outre un groupe alkyle, alcényle ou alcynyle chacun à chaîne droite ou à chaîne ramifiée, avec jusqu'à 4 atomes de carbone,

$R^4$, $R^5$, $R^6$, $R^7$ et $R^8$ représentent indépendamment les uns des autres l'hydrogène, un groupe cyano, nitro, un halogène ou un groupe alkyle, alkoxy ou alkoxycarbonyle, chacun à chaîne droite ou à chaîne ramifiée, avec dans chaque cas jusqu'à 4 atomes de carbone, en outre un groupe halogénalkyle ou halogénalkoxy, chacun à chaîne droite ou à chaîne ramifiée, avec dans chaque cas jusqu'à 4 atomes de carbone et jusqu'à 9 atomes d'halogènes identiques ou différents, ou un reste $-S(O)_n-R^{10}$,

$R^9$ représente l'hydrogène, un groupe alkyle, alcényle, alcynyle, alkoxyalkyle, alkylthioalkyle, alkoxy, alkylthio, alkylamino, dialkylamino ou halogénalkyle, chacun à chaîne droite ou à chaîne ramifiée, avec dans chaque cas jusqu'à 4 atomes de carbone dans les parties alkyle individuelles et dans le cas du groupe halogénalkyle, avec jusqu'à 9 atomes d'halogènes, identiques ou différents, en outre un groupe cycloalkyle de 3 à 7 atomes de carbone portant éventuellement un ou plusieurs substituants halogéno, alkyle inférieur ou halogénalkyle inférieur identiques ou différents, ainsi qu'un groupe phényle, phénoxy, phénoxyalkyle, phénylthio ou phénylamino, chacun portant le cas échéant un ou plusieurs substituants identiques ou différents, et on considère alors comme substituants du groupe phényle les substituants mentionnés dans le cas de $R^9$;

$R^{10}$ est un groupe amino, ainsi qu'un groupe alkyle, alkylamino, dialkylamino ou halogénalkyle, chacun à chaîne droite ou à chaîne ramifiée et avec dans chaque cas jusqu'à 4 atomes de carbone dans les parties individuelles, et dans le cas du groupe halogénalkyle, avec jusqu'à 9 atomes d'halogènes identiques ou différents,

X est l'oxygène ou le soufre,

n est le nombre 0, 1 ou 2 et

Het désigne un hétérocycle pentagonal ou hexagonal saturé ou non saturé, portant éventuellement un ou plusieurs substituants identiques ou

différents, qui comprend un à trois hétéroatomes identiques ou différents du groupe azote, oxygène et soufre et qui peut être lié par l'intermédiaire d'un atome de carbone ou d'un atome d'azote, et on considère alors comme substituants: un halogène, un groupe nitro, ainsi qu'un groupe alkyle, alkoxy, alkylthio ou halogénalkyle chacun à chaîne droite ou à chaîne ramifiée avec, dans chaque cas, jusqu'à 4 atomes de carbone et, le cas échéant, jusqu'à 9 atomes d'halogènes identiques ou différents,

caractérisé en ce que:

(a) on fait réagir des phénylhydrazines de formule (II)

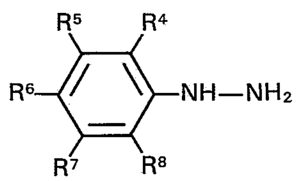

(II)

dans laquelle

$R^4$, $R^5$, $R^6$, $R^7$ et $R^8$ ont la définition indiquée ci-dessus, avec des dérivés d'acrylonitrile de formule (III),

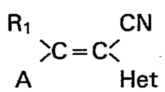

(III)

dans laquelle

$R^1$ et Het ont la définition indiquée ci-dessus et

A représente le chlore, le brome, un groupe méthoxy, éthoxy ou diméthylamino,

ou bien tout d'abord dans une première étape, le cas échéant en la présence d'un diluant et en la présence éventuelle d'une substance auxiliaire de réaction, pour former les dérivés de phénylhydrazine de formule (IV)

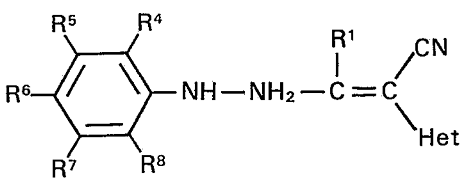

(IV)

dans laquelle

$R^1$, $R^4$, $R^5$, $R^6$, $R^7$, $R^8$ et Het ont la définition indiquée ci-dessus,

et on cyclise ces dérivés dans une seconde étape, éventuellement en présence d'un diluant et en la présence éventuelle d'un catalyseur acide, ou bien on les cyclise directement dans une seule étape réactionnelle sans isolement du stade intermédiaire (IV), le cas échéant en présence d'un diluant, en les 5-aminopyrazoles de formule (Ia)

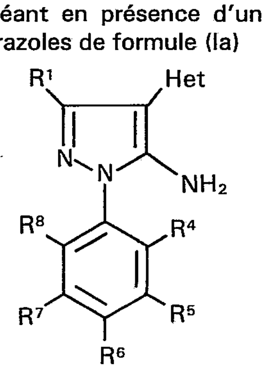

(Ia)

dans laquelle

$R^1$, $R^4$, $R^5$, $R^6$, $R^7$, $R^8$ et Het ont la définition indiquée ci-dessus, ou bien

(b) on fait réagir les 5-aminopyrazoles obtenus selon le procédé (a) de formule (Ia)

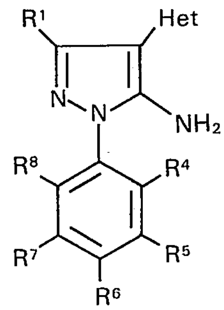

(Ia)

dans laquelle

$R^1$, $R^4$, $R^5$, $R^6$, $R^7$, $R^8$ et Het ont la définition indiquée ci-dessus,

d'une manière généralement classique avec des agents d'acylation ou des agents d'alkylation de formule (V)

$$R^{11}\text{-}A' \qquad (V)$$

dans laquelle

$R^{11}$ est un groupe alkyle, alcényle ou alcynyle, chacun à chaîne droite ou ramifiée avec jusqu'à

X

‖

4 atomes de carbone ou un reste -C-$R^9$ dans lequel X et $R^9$ ont la définition indiquée ci-dessus et

A' représente le chlore, le brome, l'iode, un groupe p-toluènesulfonyloxy, alkoxysulfonyloxy ou acyloxy,

ou bien avec des iso(thio)cyanates de formule (VI),

$$R^{12}\text{-}N=C=X \qquad (VI)$$

dans laquelle

$R^{12}$ est un groupe alkyle à chaîne droite ou à chaîne ramifiée ayant jusqu'à 4 atomes de carbone, ou un groupe phényle portant éventuellement un à trois substituants identiques ou différents, et on considère alors comme substituants: un halogène, un groupe alkyle, alkoxy ou halogénalkyle, chacun à chaîne droite ou à chaîne ramifiée et avec jusqu'à 4 atomes de carbone et, dans le cas du groupe halogénalkyle, avec jusqu'à 9 atomes d'halogènes identiques ou différents, et

X a la définition indiquée ci-dessus,

le cas échéant en présence d'un diluant et an la présence éventuelle d'un accepteur d'acide pour former les 5-aminopyrazoles alkylés ou acylés sur l'atome d'azote, de formule (Ib),

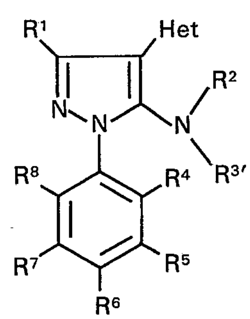

(Ib)

dans laquelle

$R^1$, $R^2$, $R^4$, $R^5$, $R^6$, $R^7$, $R^8$ et Het ont la définition indiquée ci-dessus et

$R^{3'}$ représente des restes identiques au reste $R^3$ indiqué ci-dessus à l'exception du reste hydrogène.

3. Compositions herbicides et influençant la croissance des plantes, caractérisées par une teneur en au moins un 5-amino-4-hétérocyclyl-1-phénylpyrazole de formule (I) suivant les revendications 1 et 2.

4. Utilisation de 5-amino-4-hétérocyclyl-1-phénylpyrazoles de formule (I) suivant les revendications 1 et 2 pour combattre une croissance non désirée de plantes et/ou comme substances de croissance des plantes.

5. Préparation de compositions herbicides et/ou de compositions influençant la croissance des plantes, caractérisée en ce qu'on mélange des 5-amino-4-hétérocyclyl-1-phénylpyrazoles de formule générale (I) suivant les revendications 1 et 2 avec des diluants et/ou des agents tensioactifs.

6. Dérivés d'acrylonitrile de formule (III)

$$\begin{matrix} R^1 & & CN \\ & \rangle C=C\langle & \\ A & & Het \end{matrix} \qquad (III)$$

dans laquelle

$R^1$ est l'hydrogène ou un groupe alkyle à chaîne droite ou à chaîne ramifiée ayant 1 à 8 atomes de carbone,

Het désigne un hétérocycle pentagonal ou hexagonal saturé ou non saturé, portant le cas échéant un ou plusieurs substituants identiques ou différents, qui contient un à trois hétéroatomes identiques ou différents du groupe formé par l'azote, l'oxygène et le soufre et qui peut être attaché par l'intermédiaire d'un atome de carbone ou d'azote, et on considère alors comme substituants: un halogène, un groupe nitro ainsi qu'un groupe alkyle, alkoxy, alkylthio ou halogénalkyle chacun à chaîne droite ou à chaîne ramifiée avec, dans chaque cas, jusqu'à 4 atomes de carbone et, le cas échéant, jusqu'à 9 atomes d'halogènes identiques ou différents,

A représente le chlore, le brome, un groupe méthoxy, éthoxy ou diméthylamino.

7. Procédé de préparation de dérivés d'acrylonitrile de formule (III)

$$\begin{matrix} R^1 & & CN \\ & \rangle C=C\langle & \\ A & & Het \end{matrix} \qquad (III)$$

dans laquelle

$R^1$ est l'hydrogène ou un groupe alkyle à chaîne droite ou à chaîne ramifiée ayant 1 à 8 atomes de carbone,

Het désigne un hétérocycle pentagonal ou hexagonal saturé ou non saturé, portant le cas échéant un ou plusieurs substituants identiques ou différents, qui contient un à trois hétéroatomes identiques ou différents du groupe formé par l'azote, l'oxygène ou le soufre et qui peut être attaché par l'intermédiaire d'un atome de carbone ou d'azote, et on considère alors comme substituants: un halogène, un groupe nitro ainsi qu'un groupe alkyle, alkoxy, alkylthio ou halogénalkyle chacun à chaîne droite ou à chaîne ramifiée avec, dans chaque cas jusqu'à 4 atomes de carbone et, le cas échéant, jusqu'à 9 atomes d'halogènes identiques ou différents et

A représente le chlore, le brome, un groupe méthoxy, éthoxy ou diméthylamino,

caractérisé en ce que

(1) au cas où A est un groupe méthoxy ou éthoxy, on fait réagir des dérivés d'acétonitrile de formule (VIII)

$$Het\text{-}CH_2\text{-}CN \qquad (VIII)$$

dans laquelle

Het a la définition indiquée ci-dessus,

avec des orthoesters de formule (IX)

$$\begin{matrix} & OR^{13} \\ R^1\text{-}C\langle & \!\!\!-\!\!- OR^{13} \\ & OR^{13} \end{matrix} \qquad (IX)$$

dans laquelle

$R^1$ a la définition indiquée ci-dessus et

$R^{13}$ est un groupe méthyle ou éthyle,

le cas échéant en présence d'un diluant, à des températures comprises entre +80°C et +200°C,

ou bien

(2) au cas où A est un groupe diméthylamino, on fait réagir des dérivés d'acétonitrile de formule (VIII)

$$Het\text{-}CH_2\text{-}CN$$

dans laquelle

Het a la définition indiquée ci-dessus,

avec des amido-acétals de formule (X)

$$\begin{matrix} & OR^{14} \\ R^1\text{-}C\langle & \!\!\!-\!\!- OR^{14} \\ & A'' \end{matrix} \qquad (X)$$

dans laquelle

$R^1$ a la définition indiquée ci-dessus

$R^{14}$ est un groupe méthyle ou éthyle et

A'' est un groupe diméthylamino,

le cas échéant en présence d'un diluant, à des températures comprises entre +80°C et 200°C, ou bien

(3) au cas où A est un groupe hydroxy, on fait réagir des dérivés d'acétonitrile de formule (VIII)

$$Het\text{-}CH_2\text{-}CN \qquad (VIII)$$

dans laquelle

Het a la définition indiquée ci-dessus,

avec des esters de formule (XI),

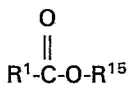

(XI)

dans laquelle
$R^1$ a la définition indiquée ci-dessus et
$R^{15}$ est un groupe méthyle ou éthyle,
le cas échéant en présence d'un diluant et en présence éventuelle d'un catalyseur basique, à des températures comprises entre 0 et 100°C,
ou bien

(4) au cas où A représente le chlore ou le brome, on fait réagir les composés hydroxyliques obtenus selon le procédé (3), de formule (IIIa)

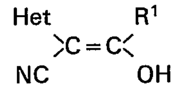

(IIIa)

dans laquelle
$R^1$ et Het ont la définition indiquée ci-dessus,
avec des agents d'halogénation, à des températures comprises entre 0°C et 100°C.